# EUROPÄISCHE PATENTSCHRIFT

(11) **EP 1 888 613 B1**
(45) Veröffentlichungstag und Bekanntmachung des Hinweises auf die Patenterteilung: **26.11.2008**
(21) Anmeldenummer: 05818005.0
(22) Anmeldetag: 08.12.2005
(51) Int. Cl.: C07H 17/00

(54) **VERFAHREN ZUR HERSTELLUNG VON THIOPHENGLYCOSIDDERIVATEN**
METHOD FOR PRODUCING THIOPHENE GLYCOSIDE DERIVATIVES
PROCÉDÉ POUR PRODUIRE DES DÉRIVÉS DE GLUCOSIDE DE THIOPHÈNE

(30) Priorität: 22.12.2004 DE 102004063099
(43) Veröffentlichungstag der Anmeldung: 20.02.2008
(73) Patentinhaber: Sanofi-Aventis Deutschland GmbH, 65929 Frankfurt am Main (DE)
(72) Erfinder: DERDAU, Volker, 65926 Frankfurt am Main (DE); BIERER, Lars, 65926 Frankfurt am Main (DE); KOSSENJANS, Michael, 65926 Frankfurt am Main (DE)
(86) Internationale Anmeldenummer: PCT/EP2005/013158
(87) Internationale Veröffentlichungsnummer: WO 2006/072334

(56) Entgegenhaltungen:
- WO-A-20/04007517

## Beschreibung

Die vorliegende Erfindung betrifft ein Verfahren zur Herstellung von Thiophenglycosidderivaten der allgemeinen Formel (I) 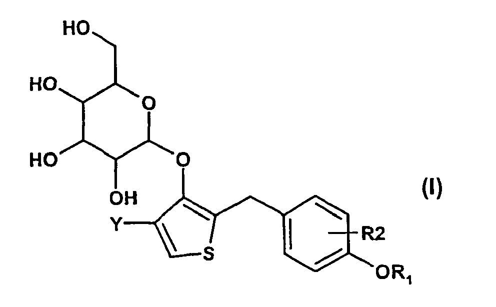 Thiophenglycosidderivate zeigen biologische Wirksamkeit, die eine Anwendung insbesondere in der Prävention und Behandlung von Diabetes Typ 1 und 2 ermöglicht.

WO2004/007517 beschreibt unter anderem verschiedene Verfahren zur Herstellung von Thiophenglycosidderivaten der allgemeinen Formel (I). Das effizienteste und kürzeste beschriebene Verfahren (B) weist jedoch bezogen auf eine technische Umsetzung verschiedene Nachteile auf. So werden die Produkte hauptsächlich per Chromatographie gereinigt. Auch die Ausbeuten sind teilweise so niedrig, dass die Abtrennung der Edukte und Nebenprodukte eine einfache Isolierung des Produktes erschwert. Bezüglich Atomökonomie wurde keine Optimierung vorgenommen. Die Verwendung von stark toxischen Verbindungen wie Natriumcyanoborhydrid, oder stark geruchsintensiven Substanzen wie Dimethylsulfid beeinträchtigen weiterhin deren Einsatz in einem technischen Prozess.

Im Hinblick auf die vorstehend geschilderten Nachteile und Probleme besteht der Bedarf, ein Verfahren bereitzustellen, das diese Nachteile und Probleme vermeidet, das sich zudem, ohne großen zusätzlichen Aufwand zu erfordern, auf einfache Weise realisieren lässt und die gewünschten Produkte mit hohem Umsatz und hoher Selektivität in hohen Ausbeuten zugänglich macht. Insbesondere hohe Ausbeuten sind eine zentrale Anforderung an das gesuchte Verfahren.

Diese Aufgabe wird überraschenderweise gelöst durch ein Verfahren zur Herstellung von Verbindungen der allgemeinen Formel (I): 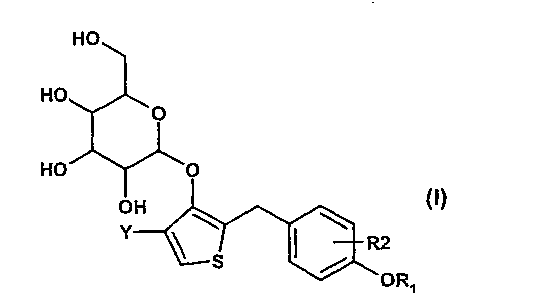 worin bedeuten
- Y: H, (C₁-C₁₀)-Alkyl;
- R1: (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
- R2: H, Cl, Br, I;
dadurch gekennzeichnet, dass man ein mehrstufiges Verfahren anwendet, worin man
**A.** Herstellung der Hydroxyketone
**A.1.** die Thiophenkomponente der Formel (II) 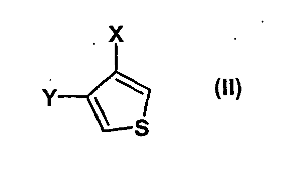 worin Y wie oben definiert ist und
X für O-(C₁-C₈)-Alkyl oder O-(C₅-C₁₀)-Aryl, wobei Aryl auch 1 bis 3 Heteroatome aus der Reihe O, N, S enthalten kann, steht;
mit einer Verbindung der Formel (III) 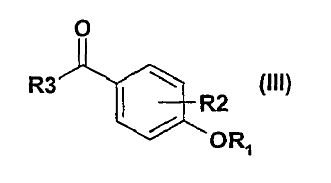 worin
- R1 und R2: wie oben definiert sind und
- R3: für Cl, Br, I steht;
in Gegenwart von 0,1 bis 10 Äquivalenten, bevorzugt 0,8 bis 1,5 Äquivalenten, einer oder mehrerer Säuren - wobei eine Säure bevorzugt ist -, bevorzugt mit Lewis-Säuren, wie SnCl₄, AlCl₃, TiCl₄, BF₃, FeCl₃, ZnCl₂, MgCl₂, ZnBr₂, MgBr₂ aber auch mit Brönsted-Säuren wie CF₃SO₃H, H₂SO₄, Toluolsulfonsäure, besonders bevorzugt mit Lewis-Säuren wie SnCl₄ oder AlCl₃, in einem geeigneten Lösungsmittel, bevorzugt in einem halogenierten Lösungsmittel, wie z. B. Dichlormethan, Chloroform, 1,2-Dichlorethan, bei - 50°C bis + 150 °C, bevorzugt bei - 20°C bis + 80 °C, besonders bevorzugt bei 5°C bis 25 °C, umsetzt zu einer Verbindung der Formel (IV), 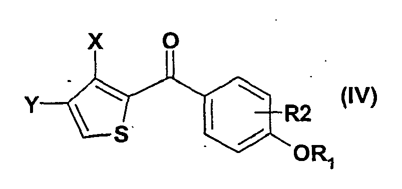 worin X, Y, R1 und R2 wie oben definiert sind; und
diese Verbindung der Formel (IV)
in Gegenwart von 0,1 bis 10 Äquivalenten, bevorzugt 0,8 bis 1,5 Äquivalenten, einer oder mehrerer Säuren - wobei eine Säure bevorzugt ist -, bevorzugt einer Lewis-Säure wie BBr₃, BCl₃, BF₃, AlCl₃, SnCl₄, TiCl₄ bei - 50°C bis + 150 °C, bevorzugt bei - 20°C bis + 80 °C, besonders bevorzugt bei 0°C bis 25 °C, zur Verbindung der Formel (IVa) 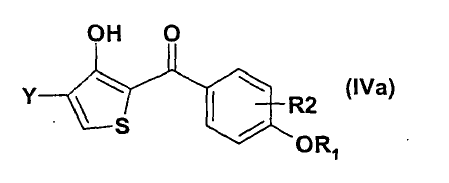 worin Y, R1 und R2 wie oben definiert sind,
umsetzt; oder
**A.2.** die Thiophenkomponente der Formel (II), 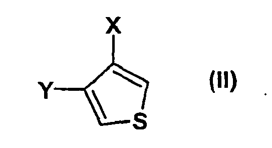 worin X und Y wie oben unter A.1. definiert sind,
   mit einer Verbindung der Formel (III) 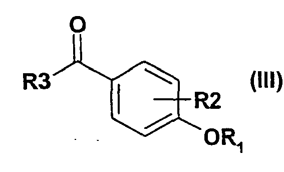 worin
   R1, R2 und R3 wie oben unter A.1, definiert sind;
   in Gegenwart von 0,1 bis 10 Äquivalenten, bevorzugt 0,8 bis 1,5 Äquivalenten, einer oder mehrerer Säuren - wobei eine Säure bevorzugt ist -, bevorzugt mit Lewis-Säuren, wie SnCl₄, AlCl₃, TiCl₄, BF₃, FeCl₃, ZnCl₂, MgCl₂ ZnBr₂, MgBr₂ aber auch Brönsted-Säuren wie CF₃SO₃H, H₂SO₄, Toluolsulfonsäure, besonders bevorzugt mit Lewis-Säuren wie SnCl₄ oder AlCl₃, in einem geeigneten Lösungsmittel, bevorzugt in einem halogenierten Lösungsmittel, wie z. B. Dichlormethan, Chloroform, 1,2-Dichlorethan, bei - 50°C bis + 150 °C, bevorzugt bei - 20°C bis + 100 °C, besonders bevorzugt bei 60°C bis 75 °C, umsetzt zu einer Verbindung der Formel (IV), 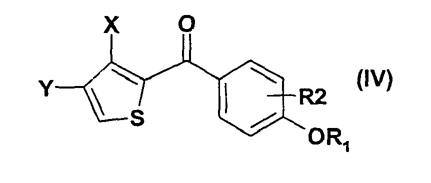 worin X, Y, R1 und R2 wie oben definiert sind; und
   diese direkt weiter in Gegenwart einer Säure, wie oben definiert, bei 0°C bis 200 °C, bevorzugt bei 20°C bis 120 °C, besonders bevorzugt bei 80 bis 90 °C, zur Verbindung der Formel (IVa) 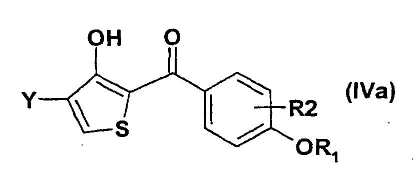 worin Y, R1 und R2 wie oben definiert sind,
   umsetzt, oder
**A.3.** die Thiophenkomponente der Formel (II) 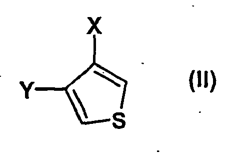 worin X und Y wie oben definiert sind,
   mit einer oder mehreren organometallischen Reagentien aus der Reihe M-(C₁-C₈)-Alkyl, MH, M-O-(C₁-C₈)-Alkyl oder M-N((C₁-C₈)-Alkyl)₂, worin M für Li, Na, K, Zn, Mg, Ca steht,
   in apolaren Lösungsmitteln, wie einem Ether, beispielsweise Diethylether, Tetrahydrofuran, Dibutylether, Dihexylether und Methyl-tert. Butylether, bei Temperaturen von -20°C bis 45°C, bevorzugt bei Temperaturen von 15°C bis 35°C, besonders bevorzugt bei 30°C bis 35°C zum reaktiven Zwischenprodukt der Formel (V) umsetzt, 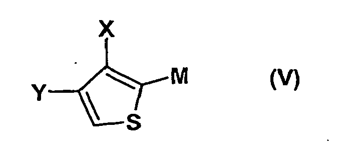 worin X, Y und M wie oben definiert sind,
   und dieses weiter mit einer Verbindung der Formel (IIIa) 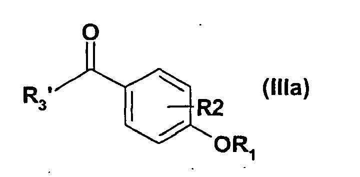 worin R1 und R2 wie oben definiert sind und
   - R3': für Cl, Br, I,
   NH-(C₁-C₈)-Alkyl, NH-O-(C₁-C₈)-Alkyl, N((C₁-C₈)-Alkyl)₂, N-(C₁-C₈)-Alkyl-O-(C₁-C₈)-Alkyl,
   N(C₃-C₈)-Cycloalkyl, wobei der Alkylring ein oder mehrere Heteroatome aus der Reihe N, O S enthalten kann,
   N((C₆-C₁₀)-Aryl)-(C₁-C₈)-Alkyl, N((C₃-C₈)-Cycloalkyl)-(C₃-C₈)-Aryl, N((C₆-C₁₀)-Aryl)₂, wobei die Aromaten bzw. die cyclischen Alkane ein oder mehrere Heteroatome aus der Reihe N, O, S enthalten können, steht,
zu einer Verbindung der Formel (IV), 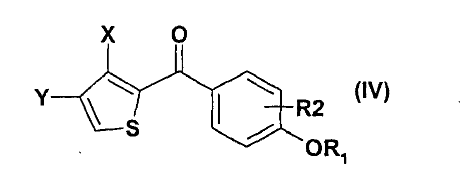 worin X, Y, R1 und R2 wie oben definiert sind; wie unter A.1. beschrieben bei Temperaturen von -20°C bis +30°C, bevorzugt -5°C bis +5°C umsetzt;
und anschließend diese Verbindung der Formel (IV) in Gegenwart einer Lewis Säure wie BBr₃, AlCl₃, SnCl₄, TiCl₄ bei 0°C bis 30 °C, bevorzugt bei 5°C bis 15°C zur Verbindung der Formel (IVa) 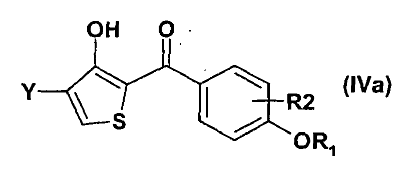 worin Y, R1 und R2 wie oben definiert sind, umsetzt;
und gegebenenfalls anschließend die Verbindungen der Formel (IVa) durch gängige Reinigungsmethoden, wie Kristallisation, Destillation oder Chromatographie, bevorzugt durch Kristallisation aus einem oder einem Gemisch mehrerer Lösungsmittel, wie Alkanen, Aromaten, halogenierten Lösungsmitteln, Ether, Ketone, Ester, Alkoholen oder Wasser, reinigt, besonders bevorzugt durch Kristallisation aus Methanol oder aus Gemischen von Dichlormethan/Heptan bzw. Methanol/Wasser oder durch Reinigung über das Natrium-Salz und - nach Neutralisation - Kristallisation aus Wasser reinigt;
und anschließend
**B.** Herstellung der Acetoglucoketone
   die Verbindung der Formel (IVa) 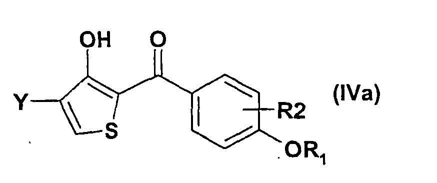 mit 0,5 bis 10 Äquivalenten, bevorzugt 1 bis 4 Äquivalenten, besonderes bevorzugt 1,5 bis 2,0 Äquivalenten eines Zuckerderivats der Formel (VI) 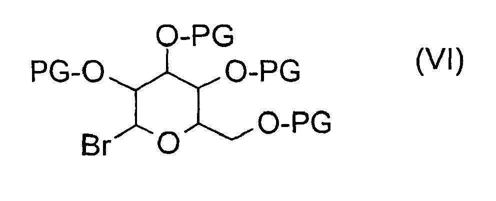 worin PG für eine OH-Schutzgruppe, wie beispielsweise Methyl, Methoxymethyl (MOM), Methylthiomethyl (MTM), Phenyldimethylsilylmethoxymethyl (SMOM), Benzyloxymethyl (BOM), p-Methoxybenzyloxymethyl (PMBM), t-Butoxymethyl, 4-Pentenyloxymethyl, 2-Methoxyethoxymethyl (MEM), 2-Trimethylsilylethoxymethyl (SEM), Trimethylsilyl (TMS), tert-Butyldimethylsilyl (TBDMS), tert-Butyldiphenylsilyl (TBDPS), Triisopropylsilyl (TIPS), oder ähnlicher Silylschutzgruppen, 1-Methyl-1-methoxyethyl (MIP), Allyl, Benzyl, Acetyl, Trifluoracetyl, Fmoc, THP, und bevorzugt für Acetyl, steht,
   in Gegenwart von 1 bis 15 Äquivalenten, bevorzugt 3 bis 6 Äquivalenten, einer organischen oder anorganischen Base, bevorzugt Kaliumcarbonat, und 0,01 bis 5 Äquivalenten, bevorzugt 0,1 bis 1 Äquivalenten, besonders bevorzugt 0,3 bis 0,6 Äquivalenten, eines Phasentransferkatalysators, bevorzugt Tetrabutylammoniumbromid oder -chlorid bzw. Benzyltributylammoniumchlorid oder - bromid, in einer Mischung aus einem organischen Lösungsmittel, bevorzugt Methylenchlorid oder 2-Methyltetrahydrofuran, und Wasser im Verhältnis 10000:1 bis 1:1, bevorzugt 500:1 bis 10:1, ganz besonders bevorzugt 200:1 bis 50:1, bei - 20°C bis + 80 °C, bevorzugt bei 5°C bis 40 °C, besonders bevorzugt bei 20°C bis 30 °C, zu der Verbindung der Formel (VII) umsetzt; 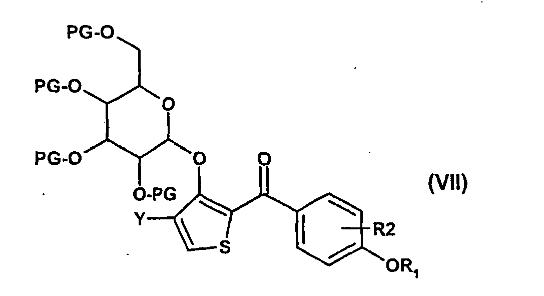 worin PG, Y, R1 und R2 wie oben definiert sind; und anschließend
**C.** Herstellung der Acetoglucomethylene
   die Verbindung der Formel (VII) wie vorstehend beschrieben,
   in einem organischen geeigneten Lösungsmittel, wie z. B. Dichlormethan, Acetonitril, Tetrahydrofuran, Dimethylformamid, DMSO und Chloroform, bevorzugt in Acetonitril mit 1 bis 15 Äquivalenten, bevorzugt 2 bis 6 Äquivalenten, eines oder mehrerer Hydriddonoren, wie z. B. Kaliumborhydrid, Natriumborhydrid, Natriumcyanoborhydrid, Triethylsilan, Triacetoxyborhydrid, bevorzugt mit Natriumcyanoborhydrid oder Natriumborhydrid, besonders bevorzugt mit Natriumborhydrid, sowie 0,1 bis 5 Äquivalenten, bevorzugt 0,5 bis 1,5 Äquivalenten, eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid, bevorzugt mit Iod, und 1 bis 25 Äquivalenten, bevorzugt 3 bis 10 Äquivalenten, eines oder mehrerer weiterer Säuren, Lewis-Säuren oder Säure-Äquivalenten, wie z.B. Trifluoressigsäure, Chlorwasserstoff, BF₃, Halogensilane, bevorzugt Chlorsilane, besonders bevorzugt Trimethylsilylchlorid bei - 100°C bis + 100 °C, bevorzugt bei - 40°C bis + 40 °C, besonders bevorzugt -15°C bis + 15 °C
   zu der Verbindung der Formel (VIII), 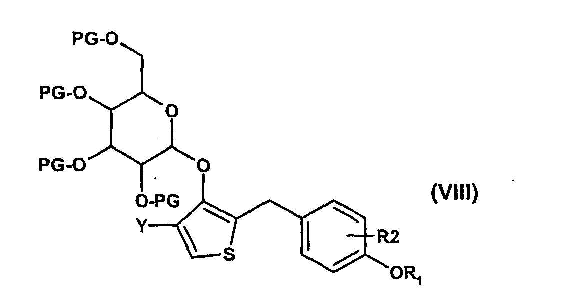 worin PG, Y, R1 und R2 wie oben definiert sind, umsetzt; anschließend
**D.** Herstellung der Thiophenglycosidderivate
   die Schutzgruppen basisch, sauer; oxidativ, reduktiv oder mit Fluorid, entsprechend den bekannten Methoden, wie z.B. in T.W. Greene, P. Wuts, Protective Groups in Organic Synthesis 1999, Wiley, New York beschrieben, abspaltet;
   bevorzugt wie vorstehend beschrieben mit PG = Acetyl, in Gegenwart von 0,01 bis 25 Äquivalenten, bevorzugt 0,05 bis 5 Äquivalenten, besonders bevorzugt 0,1 bis 0,5 Äquivalenten einer organischen oder anorganischen Base, bevorzugt, wie z. B. Natrium- oder Kaliummethanolat, Natrium- oder Kaliumhydroxid, bevorzugt Natriummethanolat, in einem geeigneten Lösungsmittel, bevorzugt Methanol, bei - 50°C bis + 150 °C, bevorzugt bei - 20 °C bis + 80 °C, besonders bevorzugt 0 °C bis 50 °C; und anschließend
   zu den Verbindungen der Formel (I) 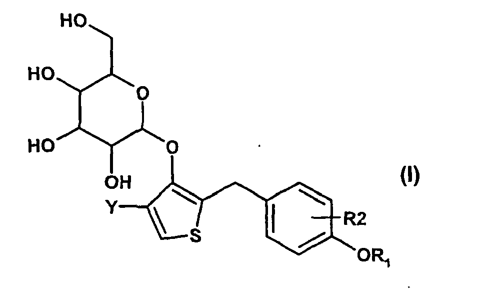
worin Y, R1 und R2 wie oben definiert sind, umsetzt;
und anschließend die Verbindungen der Formel (I) durch gängige Reinigungsmethoden, wie Kristallisation oder Chromatographie, bevorzugt durch Kristallisation aus einem oder einem Gemisch mehrerer Lösungsmittel, wie Alkanen, Aromaten, halogenierten Lösungsmitteln, Ether, Ketone, Ester, Alkoholen oder Wasser, reinigt, besonders bevorzugt durch Kristallisation aus Alkoholen oder Gemischen von Alkoholen/Wasser, ganz besonders bevorzugt durch Kristallisation aus Methanol/Wasser.
Bevorzugt ist ein mehrstufiges Verfahren zur Herstellung der Verbindungen der Formel (I), worin der Schritt **A.** Herstellung der Hydroxyketone aus den vorstehend beschriebenen Varianten **A2** oder **A3** besteht:
Verfahren zur Herstellung von Verbindungen der allgemeinen Formel (I): 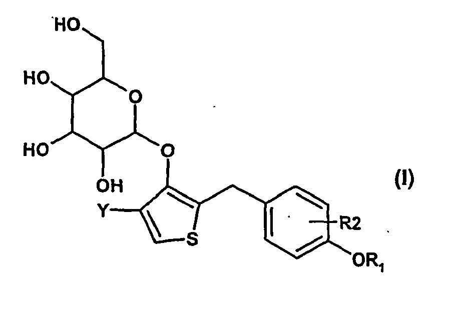
worin bedeuten
- Y: H, (C₁-C₁₀)-Alkyl;
- R1: (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
- R2: H, Cl, Br, I;
dadurch gekennzeichnet, dass man
**A.** Herstellung der Hydroxyketone
**A.2.** die Thiophenkomponente der Formel (II), 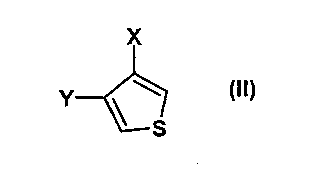 worin Y wie oben definiert ist und
   X für O-(C₁-C₈)-Alkyl oder O-(C₅-C₁₀)-Aryl, wobei Aryl auch 1 bis 3 Heteroatome aus der Reihe O, N, S enthalten kann, steht;
   mit einer Verbindung der Formel (III) 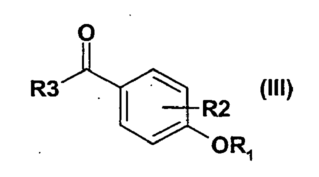 worin
   - R1 und R2: wie oben definiert ist und
   - R3: für Cl, Br, I steht;
   in Gegenwart von 0,1 bis 10 Äquivalenten einer oder mehrerer Säuren in einem geeigneten Lösungsmittel bei - 50 bis + 150 °C umsetzt zu einer Verbindung der Formel (IV), 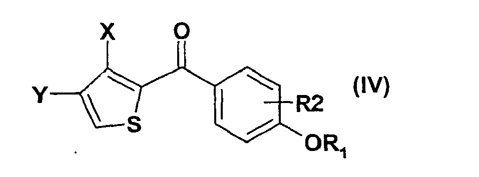 worin X, Y, R1 und R2 wie oben definiert sind; und
   diese direkt weiter in Gegenwart einer Säure wie oben definiert bei 0 bis 200 °C zur Verbindung der Formel (IVa) 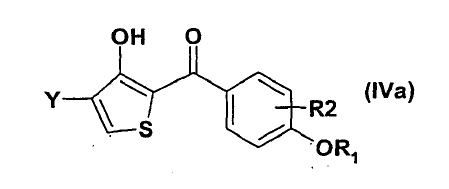 worin Y, R1 und R2 wie oben definiert sind,
   umsetzt, oder
   **A.3.** die Thiophenkomponente der Formel (II) 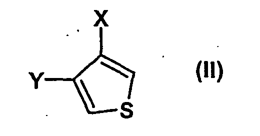 worin X und Y wie oben definiert sind,
   mit einer oder mehreren organometallischen Reagentien aus der Reihe M-(C₁-C₈)-Alkyl, MH, M-O-(C₁-C₈)-Alkyl oder M-N((C₁-C₈)-Alkyl)₂, worin M für Li, Na, K, Zn, Mg, Ca steht,
   in apolaren Lösungsmitteln bei Temperaturen von -20 bis 45°C zum reaktiven Zwischenprodukt der Formel (V) umsetzt, 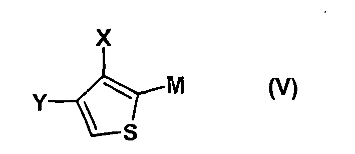 worin X, Y und M wie oben definiert sind,
   und dieses weiter mit einer Verbindung der Formel (IIIa) 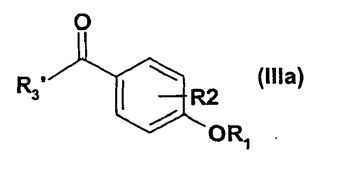 worin R1 und R2 wie oben definiert sind und
   - R3': für Cl, Br, I,
   NH-(C₁-C₈)-Alkyl-, NH-O-(C₁-C₈)-Alkyl, N((C₁-C₈)-Alkyl)₂, N-(C₁-C₈)-Alkyl-O-(C₁-C₈)-Alkyl,
   N(C₃-C₈)-Cycloalkyl, wobei der Alkylring ein oder mehrere Heteroatome aus der Reihe N, O S enthalten kann,
   N((C₆-C₁₀)-Aryl)-(C₁-C₈)-Alkyl, N((C₃-C₈)-Cycloalkyl)-(C₃-C₈)-Aryl, N((C₆-C₁₀)-Aryl)₂, wobei die Aromaten bzw. die cyclischen Alkane ein oder mehrere Heteroatome aus der Reihe N, O, S enthalten können, steht,
zu einer Verbindung der Formel (IV), 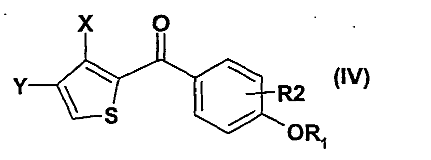 worin X, Y, R1 und R2 wie oben definiert sind; wie unter A.2. beschrieben bei Temperaturen von -20°C bis +30°C umsetzt;
und anschließend diese Verbindung der Formel (IV) in Gegenwart einer Lewis Säure zur Verbindung der Formel (IVa) 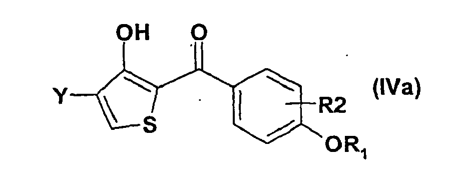 worin Y, R1 und R2 wie oben definiert sind, umsetzt und gegebenenfalls anschließend die Verbindungen der Formel (IVa) durch gängige Reinigungsmethoden reinigt;
und anschließend
**B.** Herstellung der Acetoglucoketone
   die Verbindung der Formel (IVa) 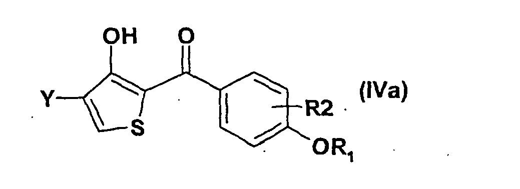 mit 0,5 bis 10 Äquivalenten eines Zuckerderivats der Formel (VI) 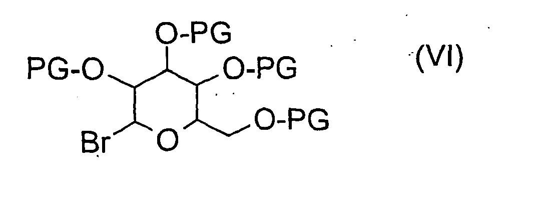 worin PG für eine OH-Schutzgruppe in Gegenwart von 1 bis 15 Äquivalenten einer organischen oder anorganischen Base und 0,01 bis 5 Äquivalenten eines Phasentransferkatalysators in einer Mischung aus einem organischen Lösungsmittel und Wasser im Verhältnis 10000:1 bis 1:1 bei - 20°C bis + 80 °C zu der Verbindung der Formel (VII) umsetzt; 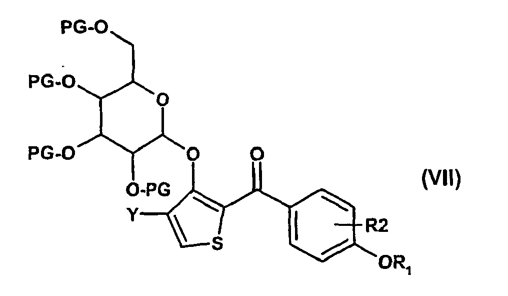 worin PG, Y, R1 und R2 wie oben definiert sind;
   und anschließend
**C**. Herstellung der Acetoglucomethylene
   die Verbindung der Formel (VII) wie vorstehend beschrieben,
   in einem organischen geeigneten Lösungsmittel mit 1 bis 15 Äquivalenten eines oder mehrerer Hydriddonoren sowie 0,1 bis 5 Äquivalenten eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid, bevorzugt mit Iod und 1 bis 25 Äquivalenten eines oder mehrerer weiterer Säuren bei - 100°C bis +100 °C zu der Verbindung der Formel (VIII) umsetzt, 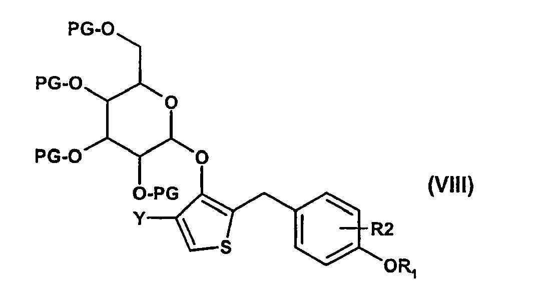 worin PG, Y, R1 und R2 wie oben definiert sind, umsetzt; anschließend
**D.** Herstellung der Thiophenglycosidderivate
   die Schutzgruppen basisch, sauer, oxidativ, reduktiv oder mit Fluorid, entsprechend den bekannten Methoden abspaltet in Gegenwart von 0,01 bis 25 Äquivalenten einer organischen oder anorganischen Base in einem geeigneten Lösungsmittel bei - 50°C bis +150 °C und anschließend
   zu den Verbindungen der Formel (I) 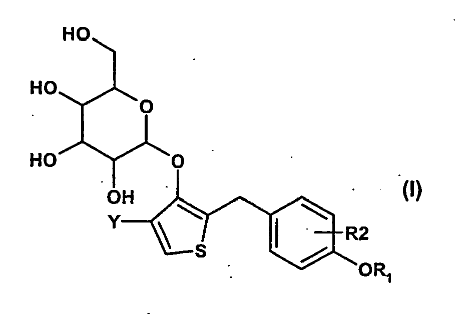 worin Y, R1 und R2 wie oben definiert sind, umsetzt
   und anschließend die Verbindungen der Formel (I) durch gängige Reinigungsmethoden reinigt.
   Die Erfindung betrifft auch ein Verfahren zur Herstellung der Zwischenverbindungen der Formel (VIII), in dem eine Verbindung der Formel (VII) 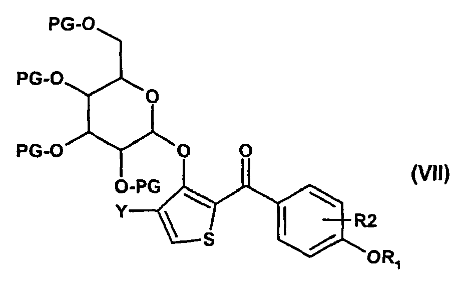 worin
   - PG: eine OH-Schutzgruppe;
   - Y: H, (C₁-C₁₀)-Alkyl;
   - R1: (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
   - R2: H, Cl, Br, I;
bedeuten,
in einem organischen geeigneten Lösungsmittel mit 1 bis 15 Äquivalenten eines oder mehrerer Hydriddonoren sowie 0,1 bis 5 Äquivalenten eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid und 1 bis 25 Äquivalenten eines oder mehrerer weiterer Säuren bei - 100°C bis +100 °C zu der Verbindung der Formel (VIII) umgesetzt wird, 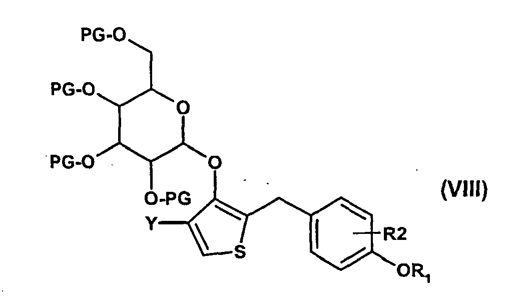 worin PG, Y, R1 und R2 wie oben definiert sind.

In einem bevorzugten Verfahren zur Herstellung der Zwischenverbindungen der Formel (VIII), wird der Aktivator Iod verwendet.

Eine weitere bevorzugte Ausführungsform ist ein Verfahren zur Herstellung der Verbindungen der Formel (I), worin bedeuten
- Y: H;
- R1: (C₁-C₄)-Alkyl, wobei ein, mehrere oder alle Wasserstoff(e) durch Fluor ersetzt sein können, bevorzugt, CH₃, C₂H_{5,}, CF₃;
- R2: H.

Die Erfindung bezieht sich auf Verbindungen der Formel (I), in Form ihrer Racemate, racemischen Mischungen und reinen Enantiomere, auf ihre Diastereomere und Mischungen davon, sowie deren Alkali-, Erdalkali-, Ammonium-, Eisen- und ähnliche, pharmakologisch verträgliche Salze.

Die Alkylreste, einschließlich Alkoxy, Alkenyl und Alkinyl, in den Substituenten R1, R3', X, Y und M können sowohl geradkettig wie verzweigt sein.

Die Zuckerreste in den Verbindungen der Formel (I) stehen sowohl für L- als auch für D-Zucker in ihrer alpha(α)- und beta(β)-Form, wie z.B. Allose, Altrose, Glucose, Mannose, Gulose, Idose, Galactose, Talose. Bevorzugt seien genannt: D-Glucose, D-Galactose, D-Allose und D-Mannose, besonders bevorzugt β-D-Glucose und β-D-Galactose, ganz besonders bevorzugt β-D-Glucose.

Das erfindungsgemäße Verfahren zeichnet sich dadurch besonders aus, dass es in hohen Ausbeuten einen technisch durchführbaren Weg zu Thiophenglycosidderivaten ermöglicht. Die alternativen Verfahren zur Herstellung der Verbindung (IV) bieten die Möglichkeit eine Vielzahl säure- oder basenlabiler Vorstufen der Verbindung (III) einzusetzen.

Die folgenden Bespiele erläutern das Verfahren, ohne es zu beschränken:

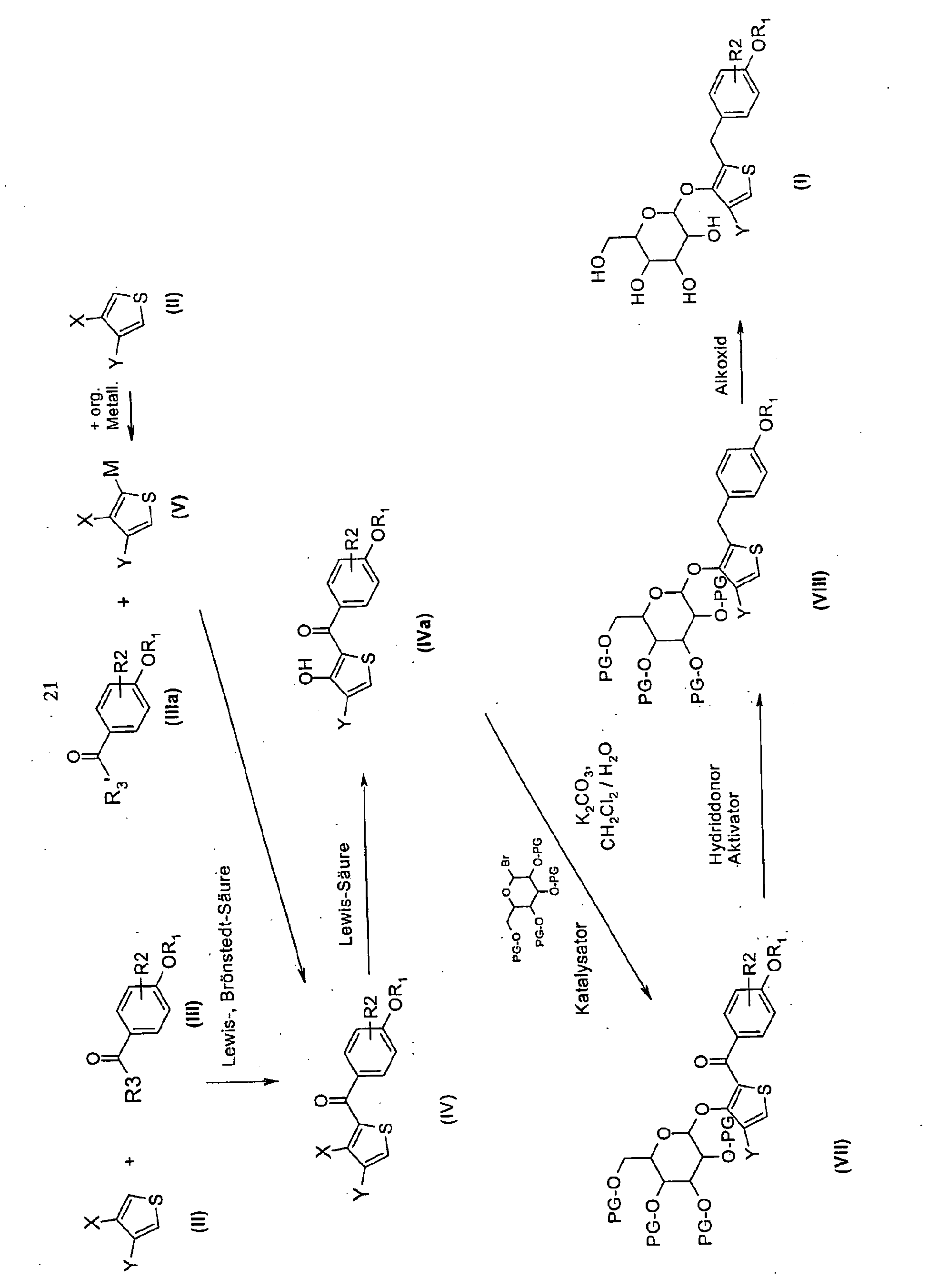

### Experimenteller Teil:

### Beispiel 1:

a) (4-Methoxy-phenyl)-(3-methoxy-thiophen-2-yl)-methanon (Variante A1)
   In einem Reaktionsgefäß werden 24.4 Gewichtsteile Zinntetrachlorid in 300 Vol.-Teilen Dichlormethan gelöst und bei 5-10°C Innentemperatur 15.0 Gewichtsteile p-Anisoylchlorid zugegeben. Dann werden 9.56 Gewichtsteile 3-Methoxythiophen bei 5-10°C Innentemperatur zugefügt und das Reaktionsgemisch bei 20-25°C für 3-5 h gerührt. Nach vollständigem Umsatz (Umsatzkontrolle) werden zum Reaktionsgemisch 135 Vol.-Teile Wasser zugegeben. Anschließend wird mit 25 Vol.-Teile 30 %ige Salzsäure gewaschen. Die organische und wässrige Phase werden getrennt, die organische Phase mit 100 Vol.-Teilen Wasser, 100 Vol.-Teilen 8%iger Natriumhydrogencarbonatlösung und 100 Vol.-Teilen Wasser gewaschen. Die organische Phase wird destillativ bis auf 40 Vol.-Teile eingeengt und bei 40°C 210 Vol.-Teile Heptan zudosiert. Die Suspension wird auf 0°C abgekühlt und der Feststoff vom Lösungsmittel befreit. Anschließend wird der hellgelbe Feststoff getrocknet. Man erhält das Produkt in 94 % Ausbeute: Smp. 98-99°C, ¹H-NMR (CDCl₃): d = 8.37 (d, J = 6.3 Hz, 1 H), 7.96 (d, J = 6.9 Hz, 2H), 6.96 (d, J = 6.9 Hz, 2H), 6.37 (d, J = 6.3 Hz, 1H), 3.91, 3.88 (s, 6H) ppm.
b) (3-Hydroxy-thiophen-2-yl-(4-methoxy-phenyl)-methanon
   Zu einer Lösung aus 1.84 Gewichtsteilen (4-Methoxy-phenyl)-(3-methoxy-thiophen-2-yl)-methanon in 25 Vol.-Teilen Dichlormethan werden bei 0-5°C 1.86 Gew.-Teile Bortribromid zugegeben und für 60 min bei 5-15°C gerührt. Es wird noch für weitere 3 h bei 20-25°C gerührt und dann 1.0 Vol.-Teile Methanol und 12 Vol.-Teile Wasser zugefügt. Mit ca. 1.4 Vol.-Teilen 33%iger Natronlauge wird ein pH-Wert von 8 eingestellt. Die Phasen werden getrennt und die organische Phase zweimal mit je 10 Vol.-Teilen Wasser gewaschen. Im Vakuum wird die organische Phase eingeengt und der Rückstand in 20 Vol.-Teilen Methanol aufgenommen. Die Lösung wird auf 60°C erwärmt und 4 Vol.-Teile Wasser zugefügt. Nach Abkühlen auf 0°C wird der ausgefallene Feststoff abgetrennt und getrocknet. Das Produkt wird als dunkelgrauer Feststoff in 91 % Ausbeute erhalten; Smp.: 86-87 °C. ¹H-NMR (DMSO-d₆): δ = 11.85 (s, 1H, OH), 7.96 (d, J = 5.4 Hz, 1 H), 7.89 (d, J = 8.8 Hz, 2H), 7.09 (d, J = 8.8 Hz, 2H), 6.91 (d, J = 5.4 Hz, 1 H), 3.85 (s, 3H) ppm.

### Beispiel 2:

### (3-Hydroxy-thiophen-2-yl)-(4-trifluormethoxy-phenyl)-methanon (Variante A2)

Zu einer Lösung aus 1.0 Gew.-Teilen Zinntetrachlorid in 10.8 Vol.-Teilen 1,2-Dichlorethan werden 0.86 Gew.-Teile 4-Trifluormethoxybenzoylchlorid zugefügt. Die Lösung wird auf 68-70°C erwärmt und bei dieser Temperatur über 2 h hinweg 0.4 Gew.-Teile 3-Methoxythiophen zugegeben. Das Reaktionsgemisch wird für 3 h bei 70°C (Umsatzkontrolle zu (IV)) und für weitere 8 h refluxiert (80-85°C, Umsatzkontrolle zu (IVa)). Bei 25°C werden 3.7 Gew.-Teile Wasser und 6.3 Vol.-Teile 30 %ige Salzsäure zugegeben. Nach Zugabe von 24 Vol.-Teilen Heptan werden die Phasen getrennt und die organische Phase mit 10 Vol.Teilen E-Wasser gewaschen. Das Lösungsmittel wird bis auf 16 Vol.-Teilen eingeengt. Man filtriert und wäscht mit Heptan. Das Filtrat wird mit 25 Vol.-Teile 0,8 %ige Natronlauge ausgerührt und die Phasen getrennt. Die Wasserphase wird mit Heptan gewaschen. Mit 7.5 %iger Salzsäure wird ein pH-Wert von 9.0 eingestellt, wobei das Produkt wieder ausfällt. Das Produkt wird abgesaugt, gewaschen und getrocknet (3-Hydroxy-thiophen-2-yl)-(4-trifluormethoxy-phenyl)-methanon wird in 53 % Ausbeute als bräunlicher bis gelblicher Feststoff isoliert. Smp.: 67-70°C; ¹H-NMR (DMSO-d6): δ = 11.45 (br s, 1 H, OH), 7.97 (d, J = 5.4 Hz, 1 H), 7.93 (d, J = 8.7 Hz, 2H), 7.51 (d, J = 8.7 Hz, 2H), 6.87 (d, J = 5.4 Hz, 1 H) ppm.

### Beispiel 3:

a) (4-Trifluormethoxy-phenyl)-(3-methoxy-thiophen-2-yl)-methanon (Variante A3)
   Zu 7 Vol.-Teilen 3-Methoxythiophen in 150 Vol.-Teilen Diethylether werden unter Schutzgasatmosphäre bei 20-25°C 8 Vol.-Teile n-BuLi (1.6 M in Hexan) zugegeben und die Lösung 30 min auf 40°C erwärmt. Das Reaktionsgemisch wird zu einer Eisgekühlten Lösung (0-5°C) aus 8.3 Gew.-Teilen N-Methoxy-N-methyl-4-trifluoromethoxy-benzamid in 100 Vol.-Teilen Diethylether gegeben. Es wird noch 1 h bei Raumtemperatur nachgerührt (Umsatzkontrolle). Es werden 50 Vol.-Teile Wasser zugegeben, die Phasen getrennt und die wässrige Phase wird 3x mit Dichlormethan extrahiert, die vereinigten organischen Phasen werden über Na₂SO₄ getrocknet und das Lösungsmittel im Vakuum entfernt. Es werden 76 % des Produktes als gelbliches Öl isoliert. ¹H-NMR (DMSO-d₆): δ = 8.04 (d, J = 5.5 Hz, 1 H), 7.82 (d, J = 8.6 Hz, 2H), 7.45 (d, J = 8.6 Hz, 2H), 7.19 (d, J = 5.5 Hz, 1 H), 3.79 (s, 3H) ppm.
b) (3-Hydroxy-thiophen-2-yl)-(4-trifluoromethoxy-phenyl)-methanon
   Zu einer Lösung aus 8.2 Gew.-Teilen BBr₃ x DMS in 500 Vol.-Teilen Dichlormethan werden bei 20-25°C 7.56 Gew.-Teile (3-Methoxy-thiophen-2-yl)-(4-trifluoromethoxyphenyl)-methanon in 100 Vol.-Teilen Dichlormethan langsam hinzugefügt. Die dunkle Lösung wird für 7 h bei 20-25°C gerührt (Umsatzkontrolle) und anschließend in einer Portion 80 Vol.-Teile gesättigte Natriumhydrogencarbonatlösung zugegeben. Die Phasen werden getrennt, die organische Phase mit 100 Vol.-Teilen Wasser gewaschen, getrocknet und das Lösungsmittel im Vakuum entfernt. Der Feststoff wird in Methanol umkristallisiert und 86 % eines hellgelben Feststoffs erhalten.

### Beispiel 4:

### Essigsäure-4,5-diacetoxy-6-acetoxymethyl-2-[2-(4-methoxy-benzoyl)-thiophen-3-yloxy]-tetrahydro-pyran-3-yl-ester

Zu einer Lösung aus 7.3 Gew.-Teilen (3-Hydroxy-thiophen-2-yl)-(4-methoxy-phenyl)-methanone in 280 Vol.-Teilen Dichlormethan werden bei 20-25°C 3.9 Gew.-Teile Benzyltributylammoniumchlorid, 19.4 Gew.-Teile Kaliumcarbonat und 2.6 Vol.-Teile Wasser hinzugefügt. Innerhalb von 2 h werden 22,5 Gew.-Teile 2,3,4,6-Tetra-O-acetylalpha-D-glucopyranosyl-bromid zugegeben. Das Reaktionsgemisch wird für 16 h bei 20-25°C gerührt (Umsatzkontrolle), Feststoffe werden abgetrennt und die organische Phase 3x mit Wasser gewaschen Die organische Phase wird eingeengt und in 95 Vol.-Teile Methanol aufgenommen. Nach der Kristallisation wird die Lösung auf 0°C abgekühlt. Der Feststoff wird abgetrennt und getrocknet. Es werden 81 % des Produktes als farbloser Feststoff erhalten; Smp.: 149 - 151 °C, 1 H-NMR (DMSO-d6): δ = 8.0 (d, 1 H), 7.7 (d, 2H), 7:1 (d, 2H), 7.0 (d, 1 H), 5.6 (d, 1 H), 5.3 (dd, 1 H), 4.9 (m, 1 H), 4.7 (dd, 1 H), 4.2 (m, 2H), 4.1 (m, 1 H), 3.8. (s, 3H, O-CH3), 2.05, 2.00, 1.90, 1.85 (s, 12H, Acetyl-CH3) ppm.

### Beispiel 5:

### Essigsäure-4,5-Diacetoxy-6-acetoxymethyl-2-[2-(4-trifluormethoxy-benzoyl)-thiophen-3- yloxy]-tetrahydro-pyran-3-yl-ester

Zu einer Lösung aus 7.1 Gew.-Teilen (3-Hydroxy-thiophen-2-yl)-(4-trifluoromethoxyphenyl)-methanon in 250 Vol.-Teilen Dichlormethan werden bei 20-25°C 3.5 Gew.-Teile Benzyltributylammoniumchlorid, 15.3 Gew.-Teile Kaliumcarbonat und 2.5 Vol.-Teile Wasser hinzugefügt. Innerhalb von 2 h werden 18,7 Gew.-Teile 2,3,4,6-Tetra-O-acetyl-alpha-D-glucopyranosyl-bromid zugegeben. Das Reaktionsgemisch wird für 16 h bei 20-25°C gerührt (Umsatzkontrolle), Feststoffe werden abgetrennt und die organische Phase 3x mit Wasser gewaschen. Die organische Phase wird eingeengt und in 100 Vol.-Teile Isopropanol aufgenommen. Bei 40-45°C werden 75 Gew.-Teile Wasser zugefügt und die Lösung auf 0°C abgekühlt. Der Feststoff wird abgetrennt und getrocknet. Es werden 90% des Produktes als farbloser Feststoff erhalten; Smp.: 90-93°C, 1 H-NMR (DMSO-d6): δ = 8.09 (d, J = 5.5 Hz, 1 H), 7.78 (d, J = 6.7 Hz, 2H), 7.43 (d, J = 6.7 Hz, 2H), 7.13 (d, J = 5.5 Hz, 1H), 5.60 (d, J = 7.9 Hz, 1H), 5.27 (dd, J = 9.5/9.5 Hz, 1 H), 4.94-4.90 (m, 1 H), 4.63 (dd, J = 9.6/9.5 Hz, 1 H), 4.21-4.17 (m, 2H), 4.06-4.04 (m, 1 H), 2.02, 1.99, 1.90, 1.84 (s, 12H, Acetyl-CH3) ppm.

### Beispiel 6:

### Essigsäure-4,5-Diacetoxy-6-acetoxymethyl-2-[2-(4-methoxy-benzyl)-thiophen-3-yloxy]-tetrahydro-pyran-3-yl-ester

Zu einer Lösung aus 10.3 Gew.-Teilen Essigsäure-4,5-diacetoxy-6-acetoxymethyl-2-[2-(4-methoxy-benzoyl)-thiophen-3- yloxy]-tetrahydro-pyran-3-yl-ester in 57 Gew.-Teilen Acetonitril wird bei -10 bis 0°C 4.5 Gew.-Teile Iod und 2.1 Gew.-Teile Natriumborhydrid (Zugabe über 60 min) sowie 11.5 Gew.-Teile Trimethylsilylchlorid (Zugabe über 45 min) zugegeben. Nach 90 min Rühren bei 0°C wird das Reaktionsgemisch mit 75 Vol.-Teilen Dichlormethan verdünnt und unter Kühlung 75 Vol.-Teile Wasser zugetropft. Nach mehrmaligem Waschen mit Wasser wird das Lösungsmittel im Vakuum entfernt und der Reststoff in 51 Vol.-Teilen Methanol aufgenommen. Das Rohprodukt wird bei 50-60°C umkristallisiert und anschließend bei -5°C abgesaugt. Der farblose Feststoff wird getrocknet und in 83 % Ausbeute erhalten. Smp.: 116-118°C; ¹H-NMR (DMSO-d₆): δ = 7.29 (d, J = 5.5 Hz, 1H), 7.09 (d, J = 6.7 Hz, 2H), 6.87 (d, J = 5.5 Hz, 1 H), 6.84 (d, J = 6.7 Hz, 2H), 5.41-5.33 (m, 2H), 5.07-4.97 (m, 2H), 4.21-4.17 (m, 2H), 4.09 (d, J = 9.7 Hz, 1H), 3.91-3.79 (m, 2H), 3.71 (s, 3H), 2.00, 1.99, 1.96, 1.95 (s, 12H, Acetyl-CH₃) ppm.

### Beispiel 7:

### Essigsäure-4,5-Diacetoxy-6-acetoxymethyl-2-[2-(4-trifluormethoxy-benzyl)-thiophen-3-yloxy]-tetrahydro-pyran-3-yl-ester

Zu einer Lösung aus 7.98 Gew.-Teilen Essigsäure-4,5-Diacetoxy-6-acetoxymethyl-2-[2-(4-trifluormethoxy-benzoyl)-thiophen-3-yloxy]-tetrahydro-pyran-3-yl-ester in 41.6 Gew.-Teilen Acetonitril wird bei -10 bis 0°C 3.24 Gew.-Teile Iod und 2.0 Gew.-Teile Natriumborhydrid (Zugabe über 60 min) sowie 11.1 Gew.-Teile. Trimethylsilylchlorid (Zugabe über 45 min) zugegeben. Nach 90 min rühren bei 0°C wird das Reaktionsgemisch mit 77 Vol.-Teile Dichlormethan verdünnt und unter Kühlung 77 Vol.-Teile Wasser zugetropft. Nach mehrmaligem Waschen mit Wasser wird das Lösungsmittel im Vakuum entfernt und der Reststoff in 35 Vol. -Teilen Methanol aufgenommen. Das Rohprodukt wird bei 40 - 50 °C umkristallisiert und anschließend bei -10 °C abgesaugt. Der farblose Feststoff wird getrocknet und 81 % eines farblosen Feststoffs erhalten. Smp.: 113-114°C; ¹H-NMR (DMSO-d₆): δ = 7.47 (d, J = 8.1 Hz, 2H), 7.40 (d, J = 5.5 Hz, 1 H), 7.30 (d, J = 8.1 Hz, 2H), 6.86 (d, J = 5.5 Hz, 1 H), 5.89 (d, J = 3.6 Hz, 1H), 5.45 (dd, J = 9.8/9.3 Hz, 1H), 5.38 (d, J = 8.0 Hz, 1H), 5.11 (dd, J = 8.0/9.8 Hz, 1H), 5.04 (dd, J = 9.3/9.3 Hz, 1H), 4.21-4.17 (m, 2H), 4.10 (dd, J = 5.0/9.8 Hz, 1H), 3.33 (s, 2H), 2.09, 2.01, 2.00, 1.99 (s, 12H, Acetyl-CH₃) ¹³C-NMR (DMSO-d₆): δ = 170.0, 169.6, 169.3, 169.3, 148.9, 147.2, 144.1, 129.5, 127.4, 123.8, 120.7, 118.9, 99.6, 71.8, 70.9, 70.8, 68.1, 66.1, 61.7, 20.4, 20.4, 20.3, 20.3 ppm.

### Beispiel 8:

### 2-Hydroxymethyl-6-[2-(4-methoxy-benzyl)-thiophen-3-yloxy]-tetrahydro-pyran-3,4,5-triol

Zu einer Suspension von 14.5 Gew.-Teilen Essigsäure-4,5-Diacetoxy-6-acetoxymethyl-2-[2-(4-methoxy-benzyl)-thiophen-3- yloxy]-tetrahydro-pyran-3-yl-ester in 91 Gew.-Teilen Methanol werden bei 0°C 0.97 Gew.-Teile Natriummethanolat (30% in Methanol) gegeben. Das Reaktionsgemisch wird für 90 min bei 0°C gerührt, dann wird mit 0.76 Gew.-Teilen Essigsäure ein pH-Wert von 7 eingestellt. Durch Zugabe von Wasser fällt das Produkt aus, und wird bei 0°C abgesaugt. Der farblose Feststoff wird getrocknet und in 83 % Ausbeute erhalten. Smp.: 154-155°C; ¹H-NMR (DMSO-d₆): δ = 7.16-7.14 (m, 3H), 6.91 (d, J = 5.5 Hz, 1 H), 6.80 (d, J = 8.6 Hz, 2H), 5.35 (s, 1 H), 5.05 (s, 1 H), 4.99 (s, 1 H), 4.63-4.53 (m, 2H), 4.01-3.97 (m, 2H), 3.71 (s, 3H), 3.66 (s, 1 H), 3.49-3.44 (m, 1 H), 3.32-3.05 (m, 4H) ppm.

### Beispiel 9:

### 2-Hydroxymethyl-6-[2-(4-trifluormethoxy-benzyl)-thiophen-3-yloxy]-tetrahydro-pyran-3,4,5-triol

Zu einer Suspension von 12.3 Gew.-Teilen Essigsäure-4,5-diacetoxy-6-acetoxymethyl-2-[2-(4-trifluormethoxy-benzyl)-thiophen-3- yloxy]-tetrahydro-pyran-3-yl-ester in 83.2 Gew.-Teilen Methanol werden bei 0°C 1.5 Gew.-Teile Natriummethanolat (30% in Methanol) gegeben. Das Reaktionsgemisch wird für 90 min bei 10°C gerührt, dann wird mit 1.58 Gew.-Teilen Essigsäure ein pH-Wert von 7 eingestellt. Durch Zugabe von Wasser fällt das Produkt aus, und bei 0°C abgesaugt. Der farblose Feststoff wird getrocknet und mit 89 % Ausbeute erhalten. Smp.: 144-145°C; ¹H-NMR (DMSO-d₆): δ = 7.41 (d, J = 8.5 Hz, 2H), 7.27 (d, J = 8.5 Hz, 2H), 7.24 (d, J = 5.5 Hz, 1H), 6.97 (d, J = 5.5 Hz, 1H), 5.37 (d, J = 4.9 Hz, 1H), 5.05 (d, J = 4.5 Hz, 1H), 4.98 (d, J = 5.3 Hz, 1H), 4.64 (d, J = 7.3 Hz, 1H), 4.56 (dd, J = 5.7/5.7 Hz, 1H), 4.12-4.04 (m, 2H), 3.72-3.68 (m, 1H), 3.51-3.47 (m, 1H), 3.32-3.12 (m, 4H); ¹⁹F-NMR (DMSO-d₆): δ = 56.8 ppm.

## Patentansprüche

1. Verfahren zur Herstellung von Verbindungen der allgemeinen Formel (I): 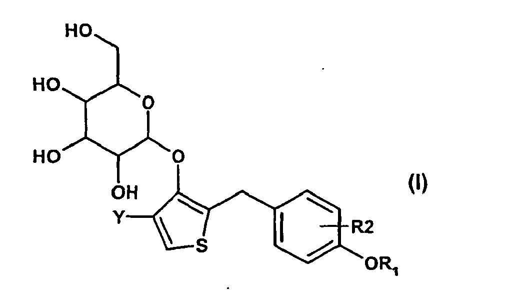 worin bedeuten
Y H, (C₁-C₁₀)-Alkyl;
R1 (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
R2 H, Cl, Br, I;
**dadurch gekennzeichnet, dass** man
**A.** Herstellung der Hydroxyketone
**A.1.** die Thiophenkomponente der Formel (II) 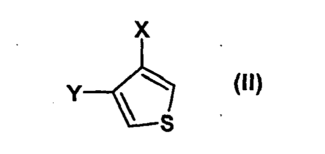 worin Y wie oben definiert ist und
X für O-(C₁-C₈)-Alkyl oder O-(C₅-C₁₀)-Aryl, wobei Aryl auch 1 bis 3 Heteroatome aus der Reihe O, N, S enthalten kann, steht;
mit einer Verbindung der Formel (III) 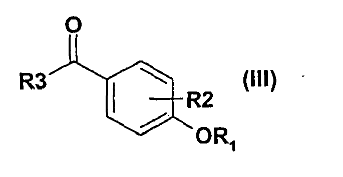 worin
R1 und R2 wie oben definiert sind und
R3 für Cl, Br, I steht;
in Gegenwart von 0,1 bis 10 Äquivalenten einer oder mehrerer Säuren in einem geeigneten Lösungsmittel bei - 50 bis + 150 °C umsetzt zu einer Verbindung der Formel (IV), 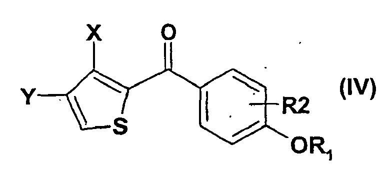 worin X, Y, R1 und R2 wie oben definiert sind; und
diese Verbindung der Formel (IV)
in Gegenwart von 0,1 bis 10 Äquivalenten einer oder mehrerer Säuren bei - 50 bis + 150 °C zur Verbindung der Formel (IVa) 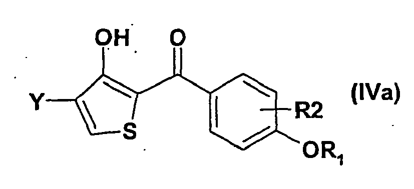 worin Y, R1 und R2 wie oben definiert sind,
umsetzt; oder
**A.2.** die Thiophenkomponente der Formel (II), 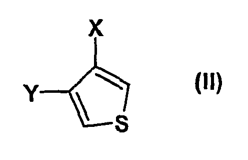 worin X und Y wie oben unter A.1. definiert sind,
mit einer Verbindung der Formel (III) 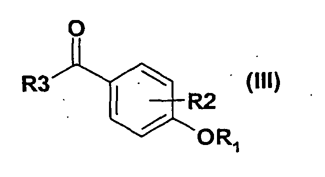 worin
R1, R2 und R3 wie oben unter A.1. definiert sind;
in Gegenwart von 0,1 bis 10 Äquivalenten einer oder mehrerer Säuren in einem geeigneten Lösungsmittel bei - 50 bis + 150 °C umsetzt zu einer Verbindung der Formel (IV), 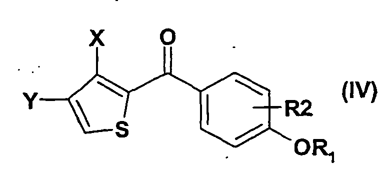 worin X, Y, R1 und R2 wie oben definiert sind; und
diese direkt weiter in Gegenwart einer Säure wie oben definiert bei 0 bis 200 °C zur Verbindung der Formel (IVa) 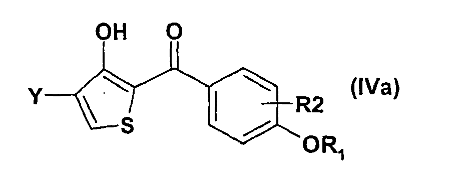 worin Y, R1 und R2 wie oben definiert sind,
umsetzt, oder
**A.3.** die Thiophenkomponente der Formel (II) 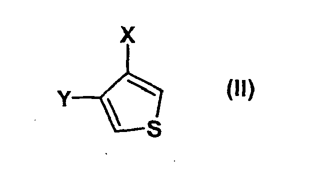 worin X und Y wie oben definiert sind,
mit einer oder mehreren organometallischen Reagentien aus der Reihe M-(C₁-C₈)-Alkyl, MH, M-O-(C₁-C₈)-Alkyl oder M-N((C₁-C₈)-Alkyl)₂, worin M für Li, Na, K, Zn, Mg, Ca steht,
in apolaren Lösungsmitteln bei Temperaturen von -20 bis 45°C zum reaktiven Zwischenprodukt der Formel (V) umsetzt, 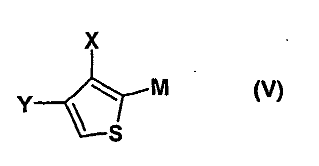 worin X, Y und M wie oben definiert sind,
und dieses weiter mit einer Verbindung der Formel (IIIa) 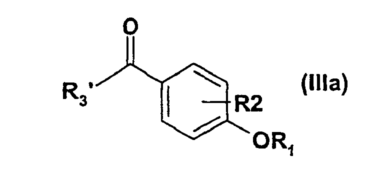 worin R1und R2 wie oben definiert sind und
R3' für Cl, Br, I,
NH-(C₁-C₈)-Alkyl, NH-O-(C₁-C₈)-Alkyl, N((C₁-C₈)-Alkyl)₂, N-(C₁-C₈)-Alkyl-O-(C₁-C₈)-Alkyl,
N(C₃-C₈)-Cycloalkyl, wobei der Alkylring ein oder mehrere Heteroatome aus der Reihe N, O, S enthalten kann,
N((C₆-C₁₀)-Aryl)-(C₁-C₈)-Alkyl, N((C₃-C₈)-Cycloalkyl)-(C₃-C₈)-Aryl, N((C₆-C₁₀)-Aryl)₂, wobei die Aromaten bzw. die cyclischen Alkane ein oder mehrere Heteroatome aus der Reihe N, O, S enthalten können, steht,
zu einer Verbindung der Formel (IV), 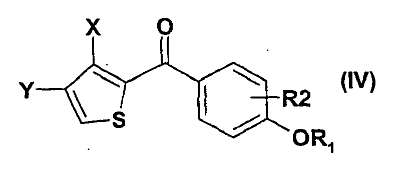 worin X, Y, R1 und R2 wie oben definiert sind; wie unter A.1. beschrieben bei Temperaturen von -20°C bis +30°C umsetzt;
und anschließend diese Verbindung der Formel (IV) in Gegenwart einer Lewis Säure zur Verbindung der Formel (IVa) 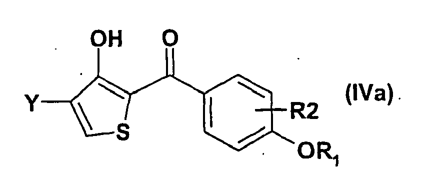 worin Y, R1 und R2 wie oben definiert sind, umsetzt und gegebenenfalls anschließend die Verbindungen der Formel (IVa) durch gängige Reinigungsmethoden reinigt;
und anschließend
**B.** Herstellung der Acetoglucoketone
die Verbindung der Formel (IVa) 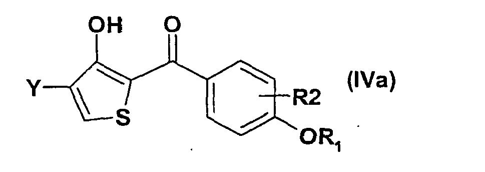 mit 0,5 bis 10 Äquivalenten eines Zuckerderivats der Formel (VI), 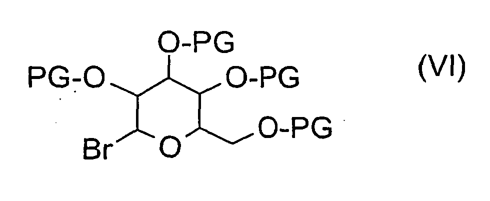 worin PG für eine OH-Schutzgruppe steht, in Gegenwart von 1 bis 15 Äquivalenten einer organischen oder anorganischen Base und 0,01 bis 5 Äquivalenten eines Phasentransferkatalysators in einer Mischung aus einem organischen Lösungsmittel und Wasser im Verhältnis 10000:1 bis 1:1 bei - 20°C bis + 80 °C zu der Verbindung der Formel (VII) umsetzt; 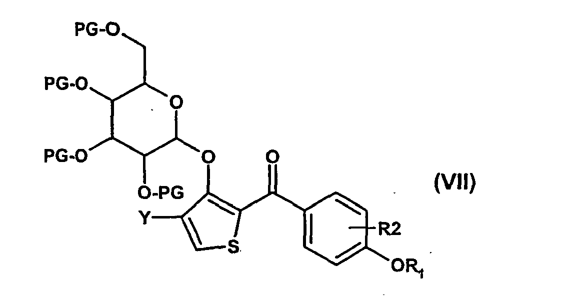 worin PG, Y, R1 und R2 wie oben definiert sind;
und anschließend
**C.** Herstellung der Acetoglucomethylene
die Verbindung der Formel (VII) wie vorstehend beschrieben,
in einem organischen geeigneten Lösungsmittel mit 1 bis 15 Äquivalenten eines oder mehrerer Hydriddonoren sowie 0,1 bis 5 Äquivalenten eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid und 1 bis 25 Äquivalenten eines oder mehrerer weiterer Säuren bei - 100°C bis +100 °C zu der Verbindung der Formel (VIII) 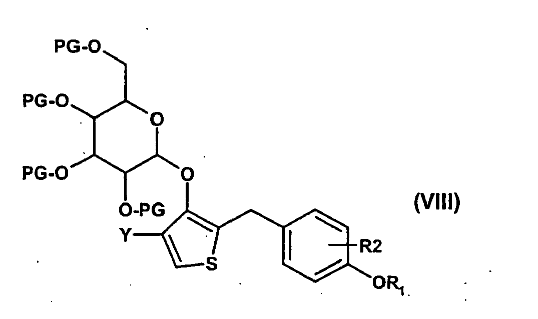 worin PG, Y, R1 und R2 wie oben definiert sind, umsetzt;
anschließend
**D.** Herstellung der Thiophenglycosidderivate
die Schutzgruppen basisch, sauer, oxidativ, reduktiv oder mit Fluorid, entsprechend den bekannten Methoden abspaltet in Gegenwart von 0,01 bis 25 Äquivalenten einer organischen oder anorganischen Base in einem geeigneten Lösungsmittel bei - 50°C bis +150 °C und anschließend
zu den Verbindungen der Formel (I) 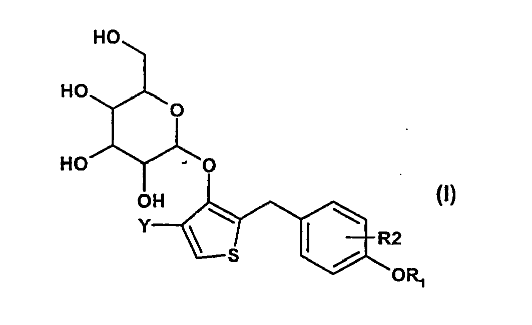
worin Y, R1 und R2 wie oben definiert sind, umsetzt
und anschließend die Verbindungen der Formel (I) durch gängige Reinigungsmethoden reinigt.

2. Verfahren zur Herstellung von Verbindungen der allgemeinen Formel (I): 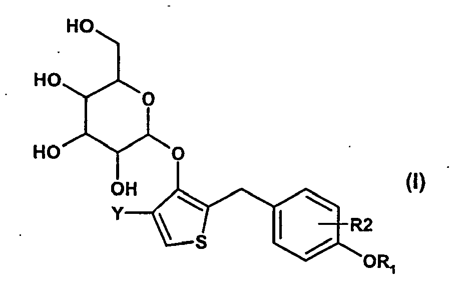 worin bedeuten
Y H, (C₁-C₁₀)-Alkyl;
R1 (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
R2 H, Cl, Br, I;
**dadurch gekennzeichnet, dass** man
**A.** Herstellung der Hydroxyketone
**A.2.** die Thiophenkomponente der Formel (II), 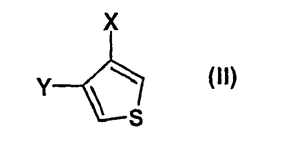 worin Y wie oben definiert ist und
X für O-(C₁-C₈)-Alkyl oder O-(C₅-C₁₀)-Aryl, wobei Aryl auch 1 bis 3 Heteroatome aus der Reihe O, N, S enthalten kann, steht;
mit einer Verbindung der Formel (III) 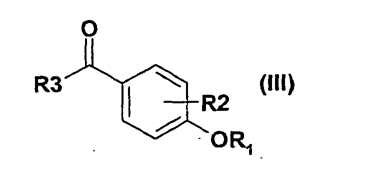 worin
R1 und R2 wie oben definiert sind und
R3 für Cl, Br, I steht;
in Gegenwart von 0,1 bis 10 Äquivalenten einer oder mehrerer Säuren in einem geeigneten Lösungsmittel bei - 50 bis + 150 °C umsetzt zu einer Verbindung der Formel (IV), 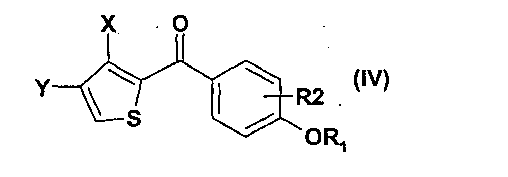 diese direkt weiter in Gegenwart einer Säure wie oben definiert bei 0 bis 200 °C zur Verbindung der Formel (IVa) 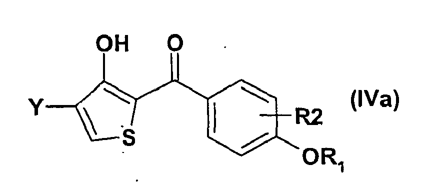 worin Y, R1 und R2 wie oben definiert sind,
umsetzt, oder
**A.3.** die Thiophenkomponente der Formel (II) 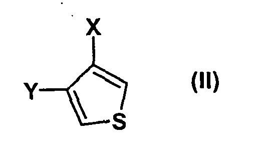 worin X und Y wie oben definiert sind,
mit einer oder mehreren organometallischen Reagentien aus der Reihe M-(C₁-C₈)-Alkyl, MH, M-O-(C₁-C₈)-Alkyl oder M-N((C₁-C₈)-Alkyl)₂, worin M für Li, Na, K, Zn, Mg, Ca steht,
in apolaren Lösungsmitteln bei Temperaturen von -20 bis 45°C zum reaktiven Zwischenprodukt der Formel (V) umsetzt, 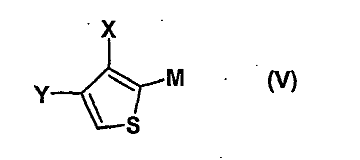 worin X, Y und M wie oben definiert sind,
und dieses weiter mit einer Verbindung der Formel (IIIa) 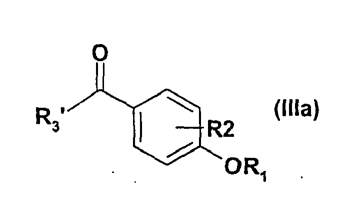 worin R1 und R2 wie oben definiert sind und
R3' für Cl, Br, I,
NH-(C₁-C₈)-Alkyl, NH-O-(C₁-C₈)-Alkyl, N((C₁-C₈)-Alkyl)₂, N-(C₁-C₈)-Alkyl-O-(C₁-C₈)-Alkyl,
N(C₃-C₈)-Cycloalkyl, wobei der Alkylring ein oder mehrere Heteroatome aus der Reihe N, O, S enthalten kann,
N((C₆-C₁₀)-Aryl)-(C₁-C₈)-Alkyl, N((C₃-C₈)-Cycloalkyl)-(C₃-C₈)-Aryl, N((C₆-C₁₀)-Aryl)₂, wobei die Aromaten bzw. die cyclischen Alkane ein oder mehrere Heteroatome aus der Reihe N, O, S enthalten können, steht,
zu einer Verbindung der Formel (IV), 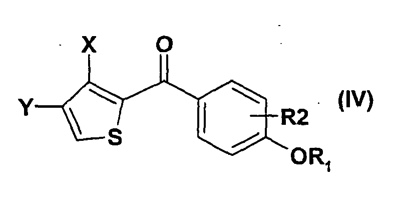 worin X, Y, R1 und R2 wie oben definiert sind; wie unter A.2. beschrieben bei Temperaturen von -20°C bis +30°C umsetzt;
und anschließend diese Verbindung der Formel (IV) in Gegenwart einer Lewis Säure zur Verbindung der Formel (IVa) 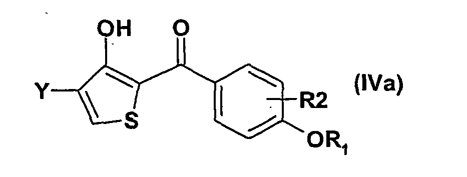 worin Y, R1 und R2 wie oben definiert sind, umsetzt und gegebenenfalls anschließend die Verbindungen der Formel (IVa) durch gängige Reinigungsmethoden reinigt;
und anschließend
**B.** Herstellung der Acetoglucoketone
die Verbindung der Formel (IVa) 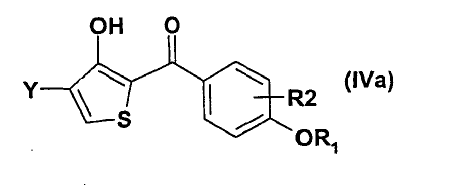 mit 0,5 bis 10 Äquivalenten eines Zuckerderivats der Formel (VI), 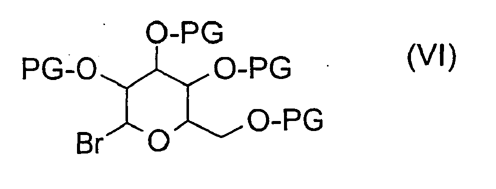 worin PG für eine OH-Schutzgruppe steht, in Gegenwart von 1 bis 15 Äquivalenten einer organischen oder anorganischen Base und 0,01 bis 5 Äquivalenten eines Phasentransferkatalysators in einer Mischung aus einem organischen Lösungsmittel und Wasser im Verhältnis 10000:1 bis 1:1 bei - 20°C bis + 80 °C zu der Verbindung der Formel (VII) umsetzt; 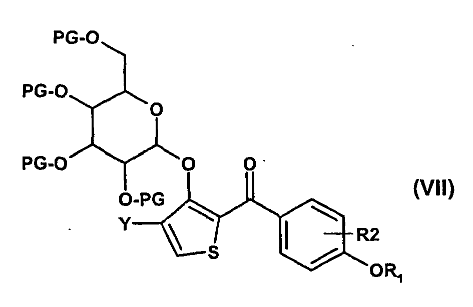 worin PG, Y, R1 und R2 wie oben definiert sind;
und anschließend
**C.** Herstellung der Acetoglucomethylene
die Verbindung der Formel (VII) wie vorstehend beschrieben,
in einem organischen geeigneten Lösungsmittel mit 1 bis 15 Äquivalenten eines oder mehrerer Hydriddonoren sowie 0,1 bis 5 Äquivalenten eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid und 1 bis 25 Äquivalenten eines oder mehrerer weiterer Säuren bei -100°C bis +100 °C zu der Verbindung der Formel (VIII), 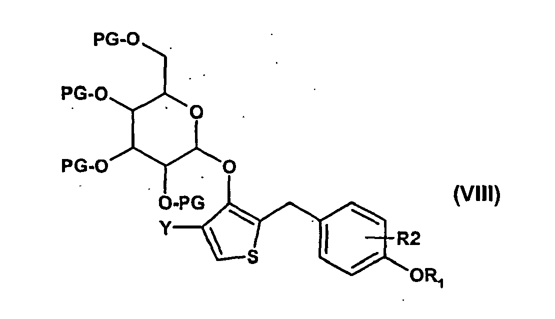 worin PG, Y, R1 und R2 wie oben definiert sind, umsetzt;
anschließend
**D.** Herstellung der Thiophenglycosidderivate
die Schutzgruppen basisch, sauer, oxidativ, reduktiv oder mit Fluorid, entsprechend den bekannten Methoden abspaltet in Gegenwart von 0,01 bis 25 Äquivalenten einer organischen oder anorganischen Base in einem geeigneten Lösungsmittel bei - 50°C bis +150 °C und anschließend
zu den Verbindungen der Formel (I) 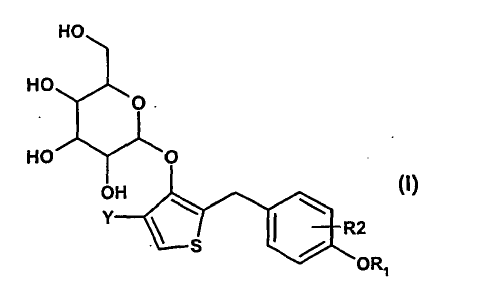 worin Y, R1 und R2 wie oben definiert sind, umsetzt
und anschließend die Verbindungen der Formel (I) durch gängige Reinigungsmethoden reinigt.

3. Verfahren zur Herstellung der Verbindungen der Formel (I) gemäß Anspruch 1 oder 2, **dadurch gekennzeichnet, dass** in Schritt C. Herstellung der Acetoglucomethylene der Aktivator Iod ist.

4. Verfahren zur Herstellung der Zwischenverbindungen der Formel (VIII), **dadurch**
**gekennzeichnet, dass**
eine Verbindung der Formel (VII), 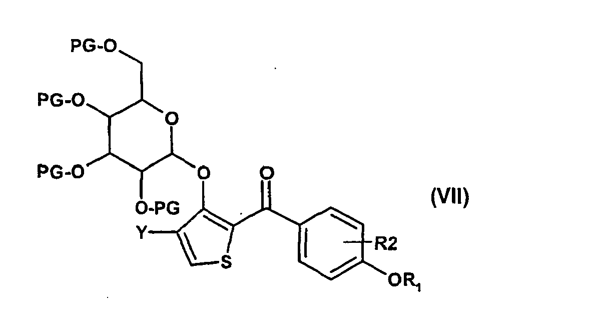 worin
PG eine OH-Schutzgruppe;
Y H, (C₁-C₁₀)-Alkyl;
R1 (C₁-C₈)-Alkyl, wobei ein, mehrere oder alle Wasserstoff(e) durch Fluor ersetzt sein können; (C₅-C₁₀)-Aryl, wobei Aryl auch 1 bis 3 Heteroatome aus der Reihe O, N, S enthalten kann;
R2 H, Cl, Br, I;
bedeuten,
in einem organischen geeigneten Lösungsmittel mit 1 bis 15 Äquivalenten eines oder mehrerer Hydriddonoren sowie 0,1 bis 5 Äquivalenten eines oder mehrerer Aktivatoren, ausgewählt aus der Gruppe Lithiumchlorid, Brom, Natrium- oder Kaliumbromid, Iod, Natrium- oder Kaliumiodid, Natrium- oder Kaliumtriiodid und 1 bis 25 Äquivalenten eines oder mehrerer weiterer Säuren bei -100°C bis +100 °C zu der Verbindung der Formel (VIII) umgesetzt wird, 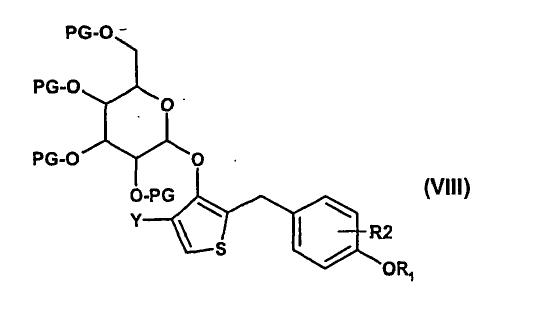 worin PG, Y, R1 und R2 wie oben definiert sind.

5. Verfahren zur Herstellung der Zwischenverbindungen der Formel (VIII), gemäß Anspruch 4, **dadurch gekennzeichnet, dass** der Aktivator Iod ist.

6. Verfahren zur Herstellung der Verbindungen der Formel (I) gemäß Anspruch 1 bis 3, worin bedeuten
Y H;
R1 (C₁-C₄)-Alkyl, wobei ein, mehrere oder alle Wasserstoff(e) durch Fluor ersetzt sein können; und
R2 H.

## Claims

1. A process for preparing compounds of the general formula (I): 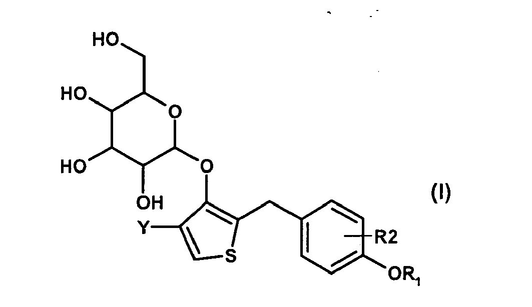 in which the meanings are
Y H, (C₁-C₁₀)-alkyl;
R1 (C₁-C₈)-alkyl, where one, more than one or all hydrogen(s) may be replaced by fluorine; (C₅-C₁₀)-aryl, where aryl may also comprise 1 to 3 heteroatoms from the series O, N, S;
R2 H, Cl, Br, I;
which comprises
A. Preparation of the hydroxy ketones
A.1. the thiophene component of the formula (II) 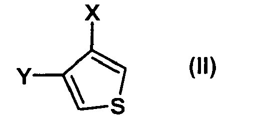 in which Y is as defined above, and
X is O-(C₁-C₈)-alkyl or O- (C₅-C₁₀)-aryl, where aryl may also comprise 1 to 3 heteroatoms from the series O, N, S;
being reacted with a compound of the formula (III) 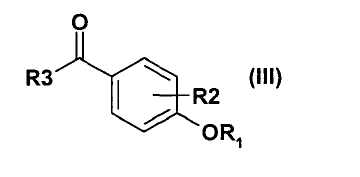 in which
R1 and R2 are as defined above, and
R3 is Cl, Br, I;
in the presence of from 0.1 to 10 equivalents of one or more acids in a suitable solvent at from - 50 to +150°C to give a compound of the formula (IV), 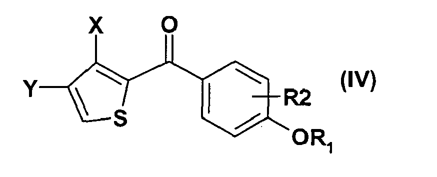 in which X, Y, R1 and R2 are as defined above; and
this compound of the formula (IV)
being converted in the presence of from 0.1 to 10 equivalents of one or more acids at from -50 to +150°C into the compound of the formula (IVa) 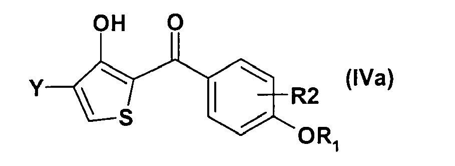 in which Y, R1 and R2 are as defined above; or
A.2. the thiophene component of the formula (II) 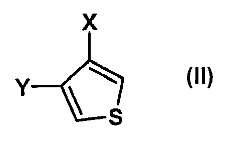 in which X and Y are as defined above under A.1.
being reacted with a compound of the formula (III) 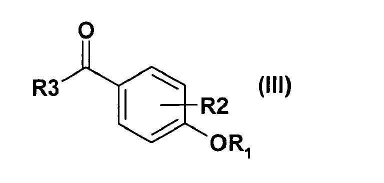 in which
R1, R2 and R3 are as defined above under A.1.;
in the presence of from 0.1 to 10 equivalents of one or more acids in a suitable solvent at from -50 to +150°C to give a compound of the formula (IV) 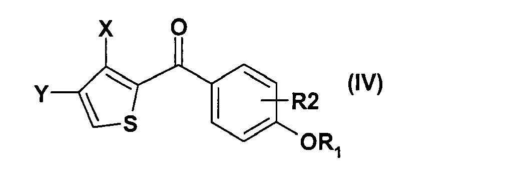 in which X, Y, R1 and R2 are as defined above; and
the latter being directly reacted further in the presence of an acid as defined above at from 0 to 200°C to give the compound of the formula (IVa) 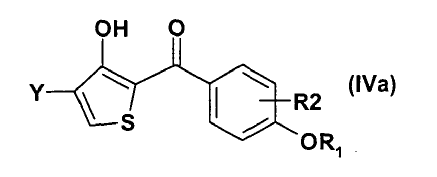 in which Y, R1 and R2 are as defined above, or
A.3. the thiophene component of the formula (II) 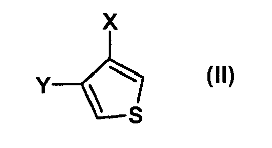 in which X and Y are as defined above,
being reacted with one or more organometallic reagents from the series M-(C₁-C₈)-alkyl, MH, M-O-(C₁-C₈)-alkyl or M-N((C₁-C₈)-alkyl)₂ in which M is Li, Na, K, Zn, Mg, Ca,
in apolar solvents at temperatures of from -20 to 45°C to give the reactive intermediate of the formula (V) 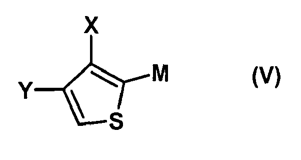 in which X, Y and M are as defined above,
and the latter being reacted further with a compound of the formula (IIIa) 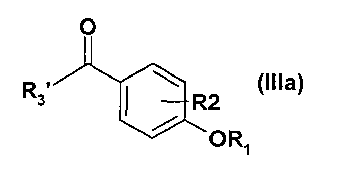 in which R1 and R2 are as defined above, and
R3' is Cl, Br, I,
NH- (C₁-C₈)-alkyl, NH-O-(C₁-C₈)-alkyl, N((C₁-C₈)-alkyl)₂, N-(C₁-C₈)-alkyl-O-(C₁-C₈)-alkyl, N(C₃-C₈)-cycloalkyl, where the alkyl ring may comprise one or more heteroatoms from the series N, O, S,
N((C₆-C₁₀)-aryl)-(C₁-C₈)-alkyl, N((C₃-C₈)-cycloalkyl)-(C₃-C₈)-aryl, N((C₆-C₁₀)-aryl)₂, where the aromatic systems and the cyclic alkanes may comprise one or more heteroatoms from the series N, O, S,
to give a compound of the formula (IV) 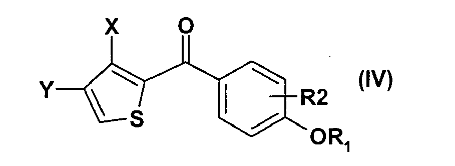 in which X, Y, R1 and R2 are as defined above; as described under A.1. at temperatures of from -20°C to +30°C;
and subsequently this compound of the formula (IV) being converted in the presence of a Lewis acid into the compound of the formula (IVa) 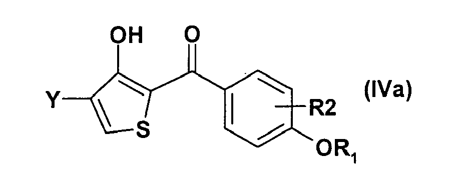 in which Y, R1 and R2 are as defined above, and where appropriate subsequently the compounds of the formula (IVa) being purified by conventional purification methods; and subsequently
B. Preparation of the acetogluco ketones
the compound of the formula (IVa) 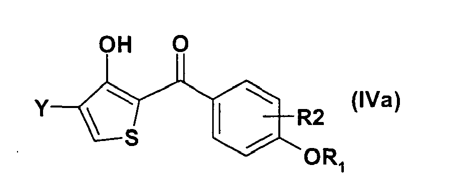 being reacted with from 0.5 to 10 equivalents of a sugar derivative of the formula (VI) 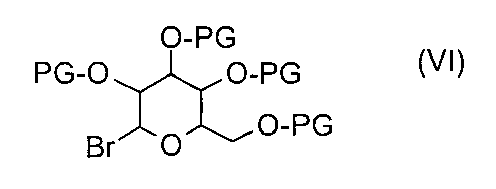 in which PG is an OH protective group, in the presence of from 1 to 15 equivalents of an organic or inorganic base and of from 0.01 to 5 equivalents of a phase-transfer catalyst in a mixture of an organic solvent and water in the ratio of from 10 000:1 to 1:1 at from -20°C to +80°C to give the compound of the formula (VII) ; 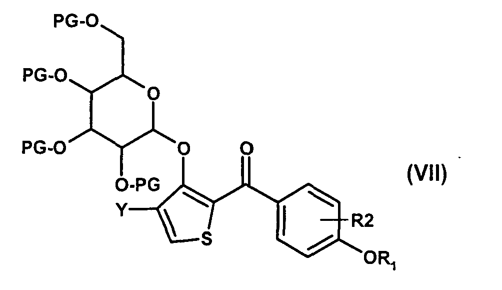 in which PG, Y, R1 and R2 are as defined above; and subsequently
C. Preparation of the acetoglucomethylenes the compound of the formula (VII) as described above being reacted
in an organic suitable solvent with from 1 to 15 equivalents of one or more hydride donors and from 0.1 to 5 equivalents of one or more activators selected from the group of lithium chloride, bromine, sodium bromide or potassium bromide, iodine, sodium iodide or potassium iodide, sodium triiodide or potassium triiodide and from 1 to 25 equivalents of one or more further acids at from -100°C to +100°C to give the compound of the formula (VIII) 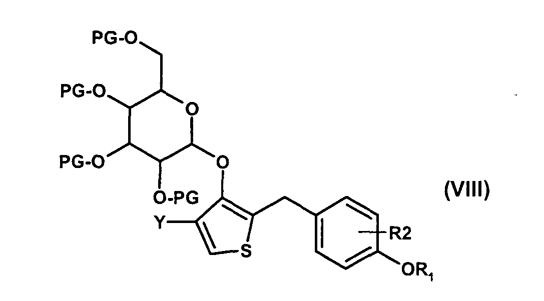 in which PG, Y, R1 and R2 are as defined above; subsequently
D. Preparation of the thiophene-glycoside derivatives
the protective groups being eliminated under basic or acidic conditions, by oxidation or reduction or with fluoride, corresponding to known methods, in the presence of from 0.01 to 25 equivalents of an organic or inorganic base in a suitable solvent at from -50°C to +150°C and subsequently
being converted into the compounds of the formula (I) 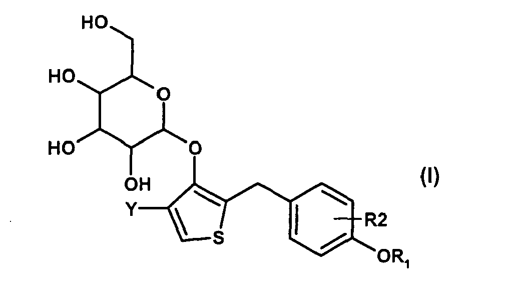
in which Y, R1 and R2 are as defined above,
and subsequently the compounds of the formula (I) being purified by conventional purification methods.

2. A process for preparing compounds of the general formula (I): 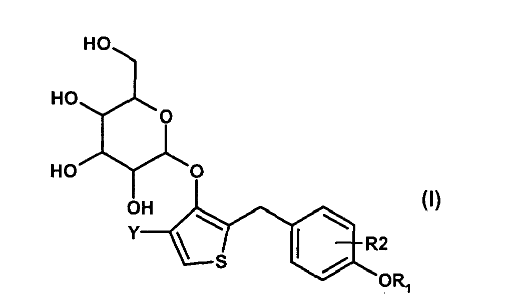 in which the meanings are
Y H, (C₁-C₁₀)-alkyl;
R1 (C₁-C₈)-alkyl, where one, more than one or all hydrogen(s) may be replaced by fluorine; (C₅-C₁₀)-aryl, where aryl may also comprise 1 to 3 heteroatoms from the series O, N, S;
R2 H, Cl, Br, I;
which comprises
A. Preparation of the hydroxy ketones
A.2. the thiophene component of the formula (II) 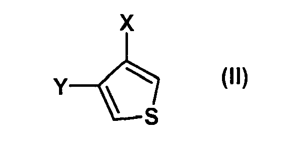 in which Y is as defined above, and
X is O-(C₁-C₈)-alkyl or O-(C₅-C₁₀)-aryl, where aryl may also comprise 1 to 3 heteroatoms from the series O, N, S;
being reacted with a compound of the formula (III) 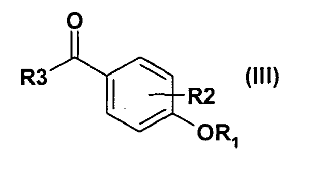 in which
R1 and R2 are as defined above, and
R3 is Cl, Br, I;
in the presence of from 0.1 to 10 equivalents of one or more acids in a suitable solvent at from - 50 to +150°C to give a compound of the formula (IV), 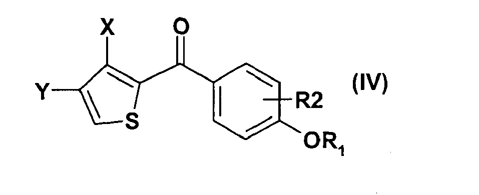 in which X, Y, R1 and R2 are as defined above; and
the latter being directly converted further in the presence of an acid as defined above at from 0 to 200°C into the compound of the formula (IVa) 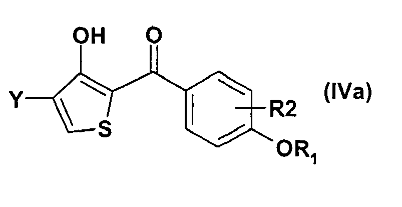 in which Y, R1 and R2 are as defined above, or
A.3. the thiophene component of the formula (II) 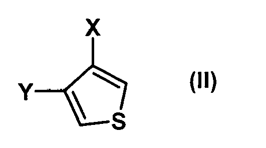 in which X and Y are as defined above,
being reacted with one or more organometallic reagents from the series M-(C₁-C₈)-alkyl, MH, M-O-(C₁-C₈)-alkyl or M-N((C₁-C₈)-alkyl)₂ in which M is Li, Na, K, Zn, Mg, Ca,
in apolar solvents at temperatures of from -20 to 45°C to give the reactive intermediate of the formula (V) 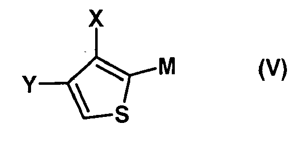 in which X, Y and M are as defined above,
and the latter being reacted further with a compound of the formula (IIIa) 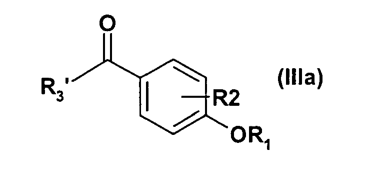 in which R1 and R2 are as defined above, and
R3' is Cl, Br, I,
NH-(C₁-C₈)-alkyl, NH-O-(C₁-C₈)-alkyl, N((C₁-C₈)-alkyl)₂, N-(C₁-C₈)-alkyl-O-(C₁-C₈)-alkyl, N(C₃-C₈)-cycloalkyl, where the alkyl ring may comprise one or more heteroatoms from the series N, O, S,
N((C₆-C₁₀)-aryl)-(C₁-C₈)-alkyl, N((C₃-C₈)-cycloalkyl)-(C₃-C₈)-aryl, N((C₆-C₁₀)-aryl)₂, where the aromatic systems and the cyclic alkanes may comprise one or more heteroatoms from the series N, O, S,
to give a compound of the formula (IV) 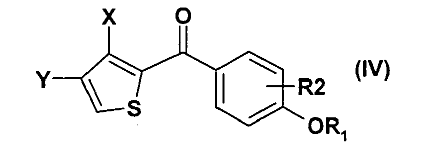 in which X, Y, R1 and R2 are as defined above; as described under A.2. at temperatures of from -20°C to +30°C;
and subsequently this compound of the formula (IV) being converted in the presence of a Lewis acid into the compound of the formula (IVa) 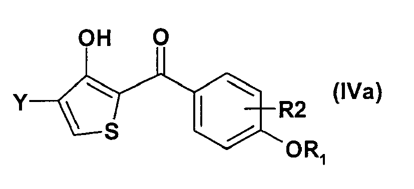 in which Y, R1 and R2 are as defined above, and where appropriate subsequently the compounds of the formula (IVa) being purified by conventional purification methods;
and subsequently
B. Preparation of the acetogluco ketones
the compound of the formula (IVa) 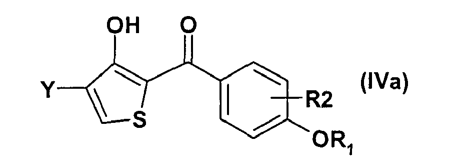 being reacted with from 0.5 to 10 equivalents of a sugar derivative of the formula (VI) 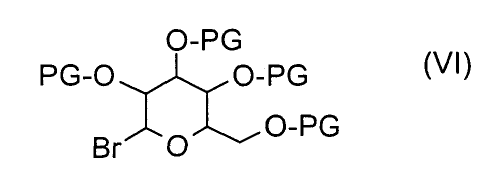 in which PG is an OH protective group, in the presence of from 1 to 15 equivalents of an organic or inorganic base and of from 0.01 to 5 equivalents of a phase-transfer catalyst in a mixture of an organic solvent and water in the ratio of from 10 000:1 to 1:1 at from -20°C to +80°C to give the compound of the formula (VII); 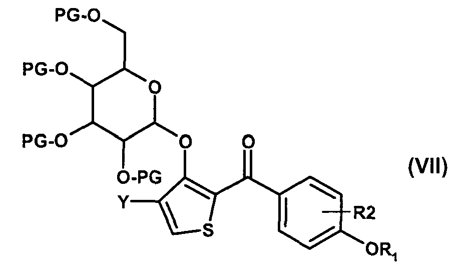 in which PG, Y, R1 and R2 are as defined above; and subsequently
C. Preparation of the acetoglucomethylenes
the compound of the formula (VII) as described above being reacted
in an organic suitable solvent with from 1 to 15 equivalents of one or more hydride donors and from 0.1 to 5 equivalents of one or more activators selected from the group of lithium chloride, bromine, sodium bromide or potassium bromide, iodine, sodium iodide or potassium iodide, sodium triiodide or potassium triiodide and from 1 to 25 equivalents of one or more further acids at from -100°C to +100°C to give the compound of the formula (VIII) 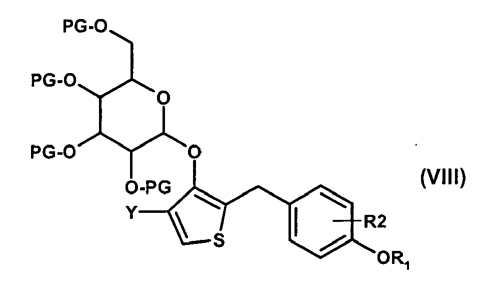 in which PG, Y, R1 and R2 are as defined above; subsequently
D. Preparation of the thiophene-glycoside derivatives
the protective groups being eliminated under basic or acidic conditions, by oxidation or reduction or with fluoride, corresponding to known methods, in the presence of from 0.01 to 25 equivalents of an organic or inorganic base in a suitable solvent at from -50°C to +150°C and subsequently
being converted into the compounds of the formula (I) 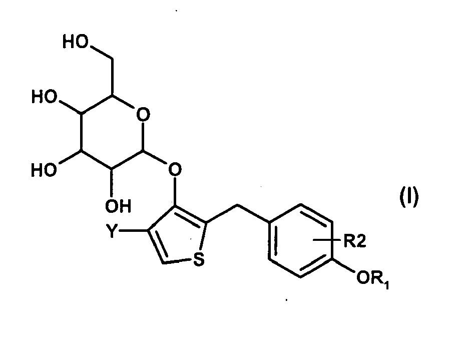 in which Y, R1 and R2 are as defined above,
and subsequently the compounds of the formula (I) being purified by conventional purification methods.

3. The process for preparing the compounds of the formula (I) as claimed in claim 1 or 2, wherein the activator in step C. Preparation of the acetoglucomethylenes is iodine.

4. A process for preparing the intermediate compounds of the formula (VIII), which comprises
a compound of the formula (VII), 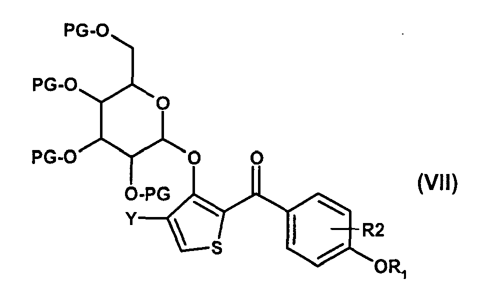 in which
PG is an OH protective group;
Y is H, (C₁-C₁₀)-alkyl;
R1 is (C₁-C₈)-alkyl, where one, more than one or all hydrogen(s) may be replaced by fluorine; (C₅-C₁₀)-aryl, where aryl may also comprise 1 to 3 heteroatoms from the series O, N, S;
R2 is H, Cl, Br, I;
being reacted in an organic suitable solvent with from 1 to 15 equivalents of one or more hydride donors and from 0.1 to 5 equivalents of one or more activators selected from the group of lithium chloride, bromine, sodium bromide or potassium bromide, iodine, sodium iodide or potassium iodide, sodium triiodide or potassium triiodide and from 1 to 25 equivalents of one or more further acids at from -100°C to +100°C to give the compound of the formula (VIII) 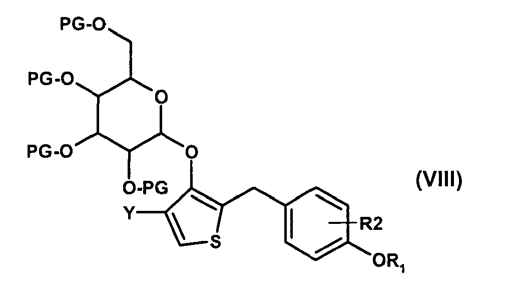 in which PG, Y, R1 and R2 are as defined above.

5. The process for preparing the intermediate compounds of the formula (VIII) as claimed in claim 4, wherein the activator is iodine.

6. The process for preparing the compounds of the formula (I) as claimed in claim 1 to 3, in which the meanings are
Y H;
R1 (C₁-C₄)-alkyl, where one, more than one or all hydrogen(s) may be replaced by fluorine; and
R2 H.

## Revendications

1. Procédé pour la préparation de composés de formule générale (I) : 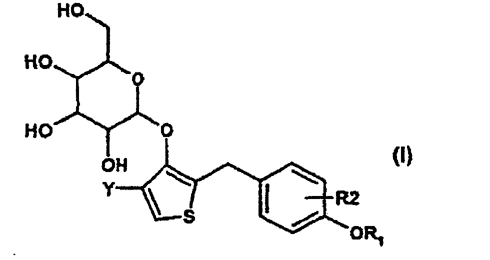 dans laquelle
Y signifie H, (C₁-C₁₀)-alkyle ;
R1 signifie (C₁-C₈)-alkyle, où un, plusieurs ou tous les hydrogènes peuvent être remplacés par fluor ; (C₅-C₁₀)-aryle, où aryle peut également contenir 1 à 3 hétéroatomes de la série O, N, S ;
R2 signifie H, Cl, Br, I ;
**caractérisé en ce qu'**on
**A.** Préparation des hydroxycétones
**A.1.** transforme le composant thiophène de formule (II) 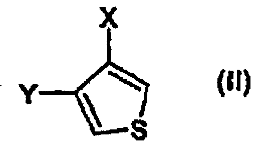 dans laquelle Y est tel que défini ci-dessus et
X représente O-(C₁-C₈)-alkyle ou O-(C₅-C₁₀)-aryle, où aryle peut également contenir 1 à 3 hétéroatomes de la série O, N, S ;
avec un composé de formule (III) 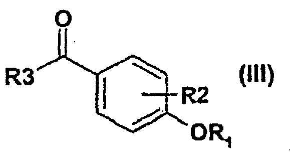 où
R1 et R2 sont tels que définis ci-dessus et
R3 représente Cl, Br, I ;
en présence de 0,1 à 10 équivalents d'un ou de plusieurs acides dans un solvant approprié à -50 jusqu'à +150°C en un composé de formule (IV), 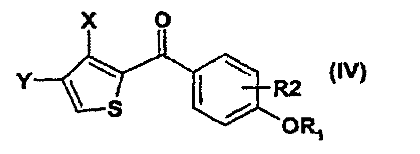 où
X, Y, R1 et R2 sont tels que définis ci-dessus ; et
on transforme ce composé de formule (IV)
en présence de 0,1 à 10 équivalents d'un ou de plusieurs acides à -50 jusqu'à +150°C en un composé de formule (IVa), 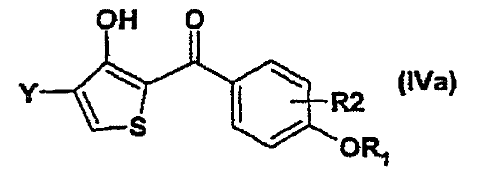 où
Y, R1 et R2 sont tels que définis ci-dessus ; ou
**A.2.** transforme le composant thiophène de formule (II) 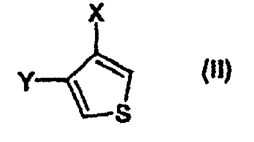 où
X et Y sont définis comme ci-dessus au point A.1.
avec un composé de formule (III) 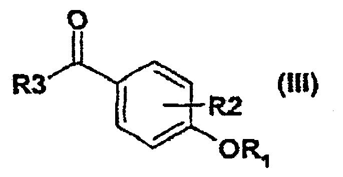 où
R1, R2 et R3 sont définis comme ci-dessus au point A.1. ;
en présence de 0,1 à 10 équivalents d'un ou de plusieurs acides dans un solvant approprié à -50 jusqu'à +150°C en un composé de formule (IV), 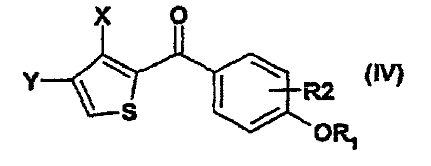 où
X, Y, R1 et R2 sont tels que définis ci-dessus ; et
on transforme celui-ci directement en présence d'un acide tel que défini ci-dessus à 0 jusqu'à 200°C en composé de formule (IVa), 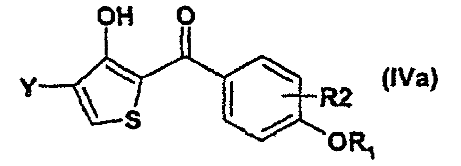 où
Y, R1 et R2 sont définis comme ci-dessus,
ou
**A.3.** transforme le composant thiophène de formule (II) 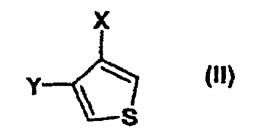 où
X et Y sont définis comme ci-dessus,
avec un ou plusieurs réactifs organométalliques de la série M-(C₁-C₈)-alkyle, MH, M-O-(C₁-C₈)-alkyle ou M-N((C₁-C₈)-alkyle)₂, où M représente Li, Na, K, Zn, Mg, Ca, dans des solvants apolaires à des températures de -20 à 45°C en produit intermédiaire réactif de formule (V), 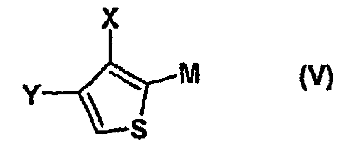 où
X, Y et M sont définis comme ci-dessus,
et on transforme celui-ci davantage avec un composé de formule (IIIa) 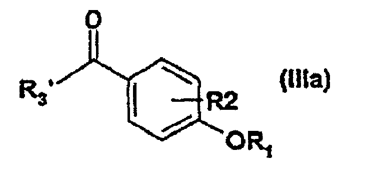 où
R1 et R2 sont tels que définis ci-dessus et
R3' représente Cl, Br, I ;
NH-(C₁-C₈)-alkyle, NH-O-(C₁-C₈)-alkyle, N((C₁-C₈)-alkyle)₂, N-(C₁-C₈)-alkyl-O-(C₁-C₈)-alkyle, N(C₃-C₈)-cycloalkyle, où le cycle alkyle peut contenir un ou plusieurs hétéroatomes de la série N, O, S,
N((C₆-C₁₀)-aryl)-(C₁-C₈)-alkyle, N((C₃-C₈)-cycloalkyl)-(C₃-C₈)-aryle, N((C₆-C₁₀)-aryle)₂, où les aromatiques ou les alcanes cycliques peuvent contenir un ou plusieurs hétéroatomes de la série N, O, S,
en un composé de formule (IV), 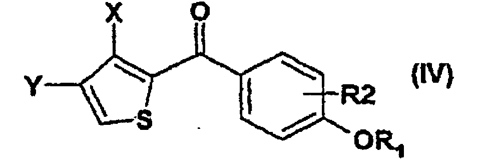 où
X, Y, R1 et R2 sont tels que définis ci-dessus ; comme décrit au point A.1. à des températures de -20°C à +30°C ;
puis on transforme ce composé de formule (IV) en présence d'un acide de Lewis en composé de formule (IVa) 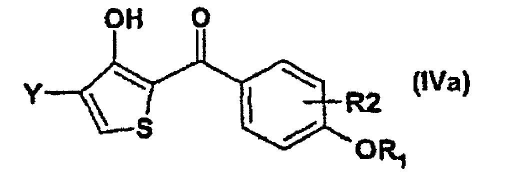 où
Y, R1 et R2 sont tels que définis ci-dessus puis on purifie le cas échéant les composés de formule (IVa) par des procédés de purification usuels ;
puis
**B.** Préparation des acétoglucocétones
on transforme le composé de formule (IVa) 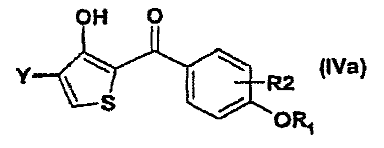 avec 0,5 à 10 équivalents d'un dérivé de sucre de formule (VI), 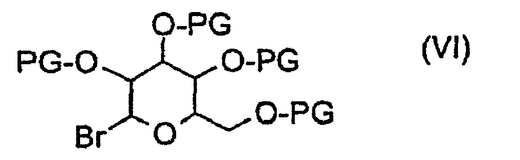 où
PG représente un groupe de protection de la fonction OH en présence de 1 à 15 équivalents d'une base organique ou inorganique et 0,01 à 5 équivalents d'un catalyseur de transfert de phases dans un mélange d'un solvant organique et d'eau dans un rapport 10 000:1 à 1:1 à -20°C jusqu'à +80°C en composé de formule (VII) ; 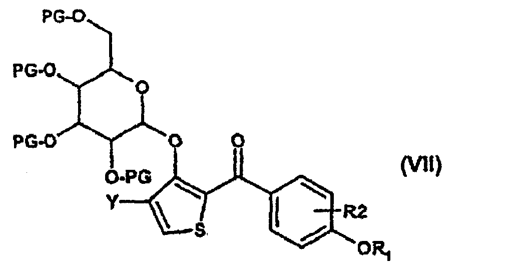
où
PG, Y, R1 et R2 sont définis comme ci-dessus ; puis
**C.** Préparation des acétoglucométhylènes
on transforme le composé de formule (VII) comme décrit ci-dessus
dans un solvant organique approprié avec 1 à 15 équivalents d'un ou de plusieurs donneurs d'hydrure ainsi que 0,1 à 5 équivalents d'un ou de plusieurs activateurs, choisis dans le groupe formé par le chlorure de lithium, le brome, le bromure de sodium ou de potassium, l'iode, l'iodure de sodium ou de potassium, le triiodure de sodium ou de potassium et 1 à 25 équivalents d'un ou de plusieurs autres acides à -100°C à +100°C en composé de formule (VIII) 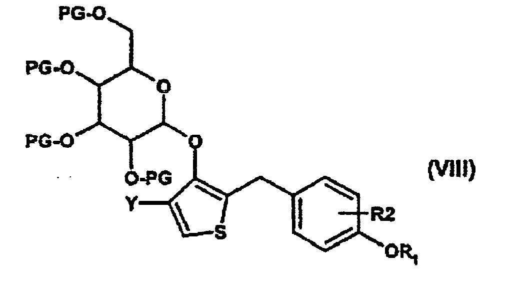 où
PG, Y, R1 et R2 sont définis comme ci-dessus ;
puis
**D.** Préparation des dérivés de thiophèneglycoside on dissocie les groupes de protection par voie basique, acide, par oxydation, par réduction ou avec du fluorure, selon les procédés connus en présence de 0,01 à 25 équivalents d'une base organique ou inorganique dans un solvant approprié à -50°C jusqu'à +150°C puis on transforme
en composés de formule (I) 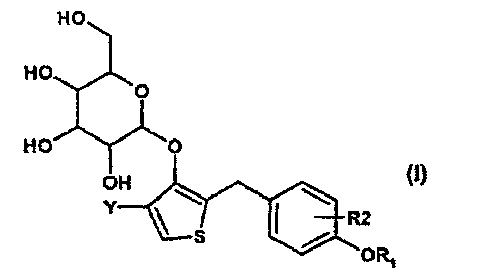
où
Y, R1 et R2 sont définis comme ci-dessus,
puis on purifie les composés de formule (I) par des procédés de purification usuels.

2. Procédé pour la préparation de composés de formule générale (I) : 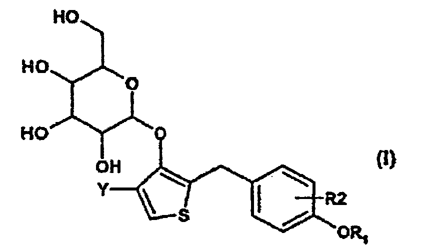 dans laquelle
Y signifie H, (C₁-C₁₀)-alkyle ;
R1 signifie (C₁-C₈)-alkyle, où un, plusieurs ou tous les hydrogènes peuvent être remplacés par fluor ; (C₅-C₁₀)-aryle, où aryle peut également contenir 1 à 3 hétéroatomes de la série O, N, S ;
R2 signifie H, Cl, Br, I ;
**caractérisé en ce qu'**on
**A.** Préparation des hydroxycétones
**A.2.** transforme le composant thiophène de formule (II) 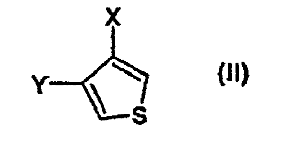 où
Y est défini comme ci-dessus et
X représente O-(C₁-C₈)-alkyle ou O-(C₅-C₁₀)-aryle, où aryle peut également contenir 1 à 3 hétéroatomes de la série O, N, S ;
avec un composé de formule (III) 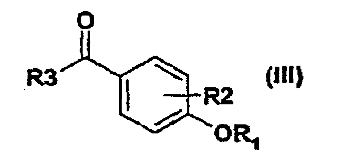 où
R1 et R2 sont tels que définis ci-dessus et
R3 représente Cl, Br, I ;
en présence de 0,1 à 10 équivalents d'un ou de plusieurs acides dans un solvant approprié à -50 à 150°C en un composé de formule (IV), 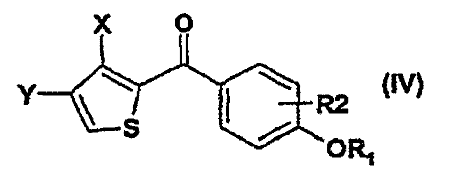 où
X, Y, R1 et R2 sont tels que définis ci-dessus ; et
on transforme celui-ci directement en présence d'un acide tel que défini ci-dessus à 0 jusqu'à 200°C en composé de formule (IVa), 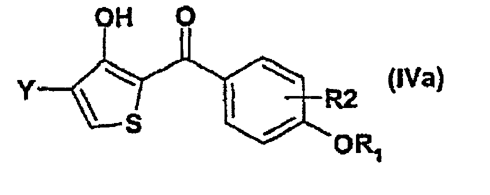 où
Y, R1 et R2 sont tels que définis ci-dessus, ou
**A.3.** transforme le composant thiophène de formule (II) 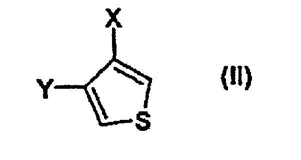
où
X et Y sont définis comme ci-dessus,
avec un ou plusieurs réactifs organométalliques de la série M-(C₁-C₈)-alkyle, MH, M-O-(C₁-C₈)-alkyle ou M-N((C₁-C₈)-alkyle)₂, où M représente Li, Na, K, Zn, Mg, Ca,
dans des solvants apolaires à des températures de -20 à 45°C en produit intermédiaire réactif de formule (V), 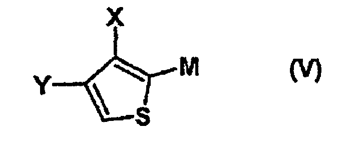 où
X, Y et M sont définis comme ci-dessus,
et on transforme celui-ci davantage avec un composé de formule (IIIa) 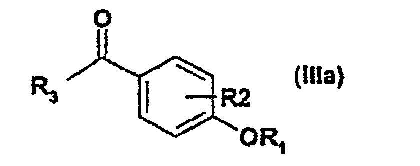 où
R1 et R2 sont tels que définis ci-dessus et
R3' représente Cl, Br, I ;
NH-(C₁-C₈) -alkyle, NH-O-(C₁-C₈)-alkyle, N((C₁-C₈)-alkyle)₂, N-(C₁-C₈)-alkyl-O-(C₁-C₈)-alkyle,
N(C₃-C₈)-cycloalkyle, où le cycle alkyle peut contenir un ou plusieurs hétéroatomes de la série N, O, S,
N((C₆-C₁₀)-aryl)-(C₁-C₈)-alkyle, N((C₃-C₈)-cycloalkyl) - (C₃-C₈) -aryle, N((C₆-C₁₀)-aryle)₂, où les aromatiques ou les alcanes cycliques peuvent contenir un ou plusieurs hétéroatomes de la série
N, O, S,
en un composé de formule (IV), 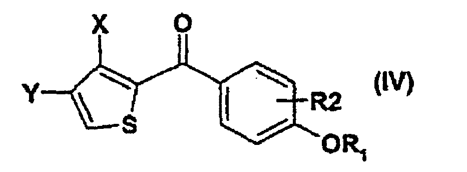 où
X, Y, R1 et R2 sont tels que définis ci-dessus ; comme décrit au point A.2. à des températures de -20°C à +30°C ;
puis on transforme ce composé de formule (IV) en présence d'un acide de Lewis en composé de formule (IVa) 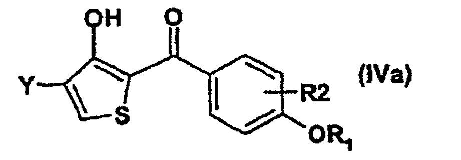 où
Y, R1 et R2 sont tels que définis ci-dessus puis on purifie le cas échéant les composés de formule (IVa) par des procédés de purification usuels ;
puis
**B.** Préparation des acétoglucocétones
on transforme ce composé de formule (IVa) 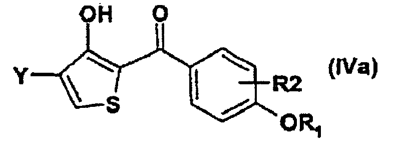 avec 0,5 à 10 équivalents d'un dérivé de sucre de formule (VI), 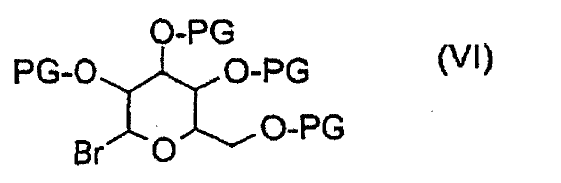 où
PG représente un groupe de protection de la fonction OH en présence de 1 à 15 équivalents d'une base organique ou inorganique et 0,01 à 5 équivalents d'un catalyseur de transfert de phases dans un mélange d'un solvant organique et d'eau dans un rapport 10 000:1 à 1:1 à -20°C jusqu'à +80°C en composé de formule (VII) ; 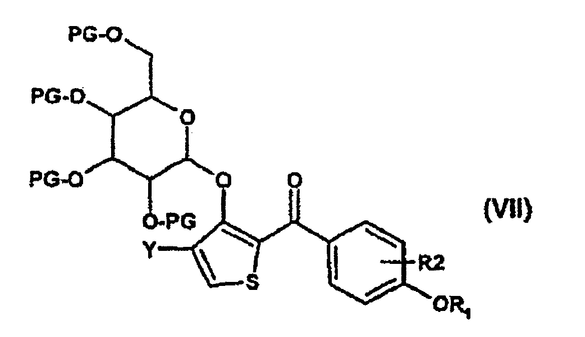
où
PG, Y, R1 et R2 sont définis comme ci-dessus ; puis
**C.** Préparation des acétoglucométhylènes
on transforme le composé de formule (VII) comme décrit ci-dessus
dans un solvant organique approprié avec 1 à 15
équivalents d'un ou de plusieurs donneurs d'hydrure ainsi que 0,1 à 5 équivalents d'un ou de plusieurs activateurs, choisis dans le groupe formé par le chlorure de lithium, le brome, le bromure de sodium ou de potassium, l'iode, l'iodure de sodium ou de potassium, le triiodure de sodium ou de potassium et 1 à 25 équivalents d'un ou de plusieurs autres acides à
- 100°C jusqu'à +100°C en composé de formule (VIII), 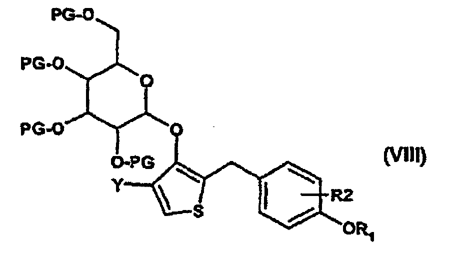
où
PG, Y, R1 et R2 sont définis comme ci-dessus ; puis
**D.** Préparation des dérivés de thiophèneglycoside on dissocie les groupes de protection par voie basique, acide, par oxydation, par réduction ou avec du fluorure, selon les procédés connus en présence de 0,01 à 25 équivalents d'une base organique ou inorganique dans un solvant approprié à -50°C jusqu'à +150°C puis on transforme
en composés de formule (I) 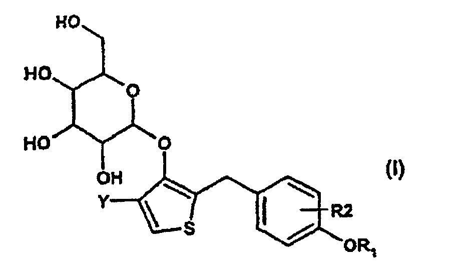
où
Y, R1 et R2 sont définis comme ci-dessus, puis on purifie les composés de formule (I) par des procédés de purification usuels.

3. Procédé pour la préparation des composés de formule (I) selon la revendication 1 ou 2, **caractérisé en ce que** dans l'étape **C.** Préparation des acétoglucométhylènes, l'activateur est l'iode.

4. Procédé pour la préparation des composés intermédiaires de formule (VIII), **caractérisé en ce qu'**on transforme un composé de formule (VII), 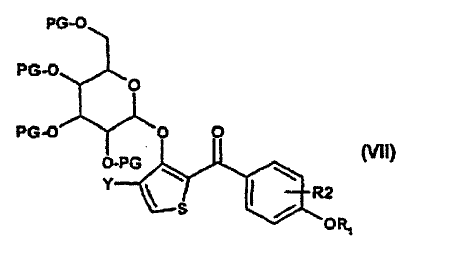 où
PG représente un groupe de protection de la fonction OH ;
Y signifie H, (C₁-C₁₀)-alkyle ;
R1 signifie (C₁-C₈)-alkyle, où un, plusieurs ou tous les hydrogènes peuvent être remplacés par fluor ; (C₅-C₁₀)-aryle, où aryle peut également contenir 1 à 3 hétéroatomes de la série O, N, S ;
R2 signifie H, Cl, Br, I ;
dans un solvant organique approprié avec 1 à 15 équivalents d'un ou de plusieurs donneurs d'hydrure ainsi que 0,1 à 5 équivalents d'un ou de plusieurs activateurs, choisis dans le groupe formé par le chlorure de lithium, le brome, le bromure de sodium ou de potassium, l'iode, l'iodure de sodium ou de potassium, le triiodure de sodium ou de potassium et 1 à 25 équivalents d'un ou de plusieurs autres acides jusqu'à -100°C à +100°C en composé de formule (VIII), 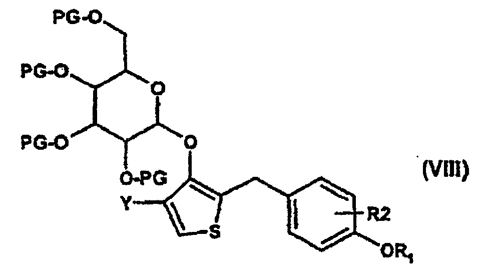 où
PG, Y, R1 et R2 sont définis comme ci-dessus.

5. Procédé pour la préparation des composés intermédiaires de formule (VIII) selon la revendication 4, **caractérisé en ce que** l'activateur est l'iode.

6. Procédé pour la préparation des composés de formule (I) selon la revendication 1 à 3, où
Y signifie H ;
R1 signifie (C₁-C₄)-alkyle, où un, plusieurs ou tous les hydrogènes peuvent être remplacés par fluor ; et
R2 signifie H.
